# EUROPEAN PATENT SPECIFICATION

(11) **EP 1 171 159 B1**
(45) Date of publication and mention of the grant of the patent: **14.05.2008**
(21) Application number: 00940231.4
(22) Date of filing: 17.04.2000
(51) Int. Cl.: A61K 39/295, A61P 31/04, A61P 31/14

(54) **COMBINATION VACCINE AGAINST STREPTOCOCCUS PNEUMONIAE AND RESPIRATORY SYNCYTIAL VIRUS (RSV)**
KOMBINATIONSIMPFSTOFF GEGEN STREPTOKOKKUS PNEUMONIAE UND RESPIRATORY SYNCYTIAL VIRUS (RSV)
VACCIN COMBINE CONTRE LE STREPTOCOCCUS PNEUMONIAE ET LE VIRUS SYNCYTIAL RESPIRATOIRE (RSV)

(30) Priority: 20.04.1999 GB 9909077
(43) Date of publication of application: 16.01.2002
(60) Divisional application: 08151478.8
(73) Proprietor: GlaxoSmithKline Biologicals s.a., 1330 Rixensart (BE)
(72) Inventor: DESCHAMPS, Marguerite, B-1330 Rixensart (BE); LAFERRIERE, Craig, Antony, Joseph, B-1330 Rixensart (BE)
(74) Representative: Crepin, Carine Marie Blanche
(86) International application number: PCT/EP2000/003514
(87) International publication number: WO 2000/062801

(56) References cited:
- WO-A-96/32963
- WO-A-96/33739
- WO-A-98/18819
- WO-A-98/51339
- US-A- 5 750 110
- US-A- 5 837 250
- LEE CHI-JEN ET AL: "Immunologic epitope, gene, and immunity involved in pneumococcal glycoconjugate." CRITICAL REVIEWS IN MICROBIOLOGY, vol. 23, no. 2, 1997, pages 121-142, XP000946772 ISSN: 1040-841X
- SCHNEERSON R ET AL: "EVALUATION OF MONOPHOSPHORYL LIPID A MPL AS AN ADJUVANT ENHANCEMENT OF THE SERUM ANTIBODY RESPONSE IN MICE TO POLYSACCHARIDE-PROTEIN CONJUGATES BY CONCURRENT INJECTION WITH MPL" JOURNAL OF IMMUNOLOGY, vol. 147, no. 7, 1991, pages 2136-2140, XP002149961 ISSN: 0022-1767
- FLETCHER T J ET AL: "Simultaneous immunisation with influenza vaccine and pneumococcal polysaccharide vaccine in patients with chronic respiratory disease." BMJ, vol. 314, no. 7095, 1997, pages 1663-1665, XP000952814 cited in the application
- GIEBINK G SCOTT: "Immunology: Promise of new vaccines." PEDIATRIC INFECTIOUS DISEASE JOURNAL, vol. 13, no. 11, 1994, pages 1064-1068, XP000952829 ISSN: 0891-3668

## Description

This invention relates to vaccine formulations, methods for preparing them and their use in prophylaxis and therapy. In particular the present invention relates to combination vaccines for administration to children and the elderly. More particularly the invention relates to combination vaccines for protection against *Streptococcus pneumoniae* and Respiratory Syncytial Virus (RSV) infection or disease.

*Streptococcus pneumoniae* is a gram positive bacteria responsible for considerable morbidity and mortality, particularly in the young and aged. Expansive colonisation of the respiratory tract, and middle ear, especially in young children, is the single most common cause for hospital visits in the US. The bacteria may become invasive, infecting the lower lungs and causing pneumonia. The rate of pneumococcal pneumonia in the US for persons over 60 years of age is estimated to be 3 to 8 per 100,000. In 20% of cases this leads to bacteremia, and other manifestations such as meningitis, with a mortality rate close to 30% even with antibiotic treatment. There are 90 known serotypes of *Streptococcus pneumoniae* which are determined by the structures of the capsular polysaccharide surrounding the bacteria, and this is its major virulence factor.

A 17 - valent pneumococcal vaccine (Moniarix) is known, based on the purified polysaccharides of the pneumococcal serotypes most commonly involved in invasive disease. The method of purification of these polysaccharides was disclosed in European Patent 72513 B1. Vaccine efficacy trials with lower valent vaccines demonstrated a 70 to 90% efficacy with respect to serotypes present in the combination. Case controlled studies in the US in persons >55 years using a 14 valent vaccine demonstrated 70% efficacy (Mills, O.F., and Rhoads, G.G; J. Clin. Epidemiol. (1996); Vol.49(6) 631-636). Inclusion of additional polysaccharides (to make a 23-valent pneumococcal vaccine) were accepted on the basis of an adequate serological response, even though there was clinical efficacy data lacking (K. R. Brown In 'Combined. Vaccines and Simultaneous Administration', Ed. Williams et al. New York Academy of Sciences 1995 pp 241-249).

Simultaneous immunisation with a 23-valent pneumococcal vaccine (Pnu-Immune 23 - Lederle USA) and influenza vaccine (Fluarix) has been studied (TJ Fletcher, TW Tunnicliffe, K Hammond, K Roberts, JG Ayres; (1997) Br. Med. J. 314: 1663) and no significant immunological differences were noted between simultaneous immunisation, or immunisations one month apart.

Pneumococcal polysaccharides can be rendered more immunogenic in infants by chemically coupling them to protein carriers. Clinical efficacy trials are being performed to verify this concept for efficacy in preventing infant Otitis media.

Infants primed with a 7 to 11-valent conjugate pneumococcal vaccine may be boosted with the 23-valent plain polysaccharide pneumococcal vaccine in order to increase the serotype coverage and IgG concentrations (Block, SL, JA. Hedrick, R.A. Smith, R.D. Tyler, M. Giordani, M.D. Blum, J. Sadoff, E. Keegan; Abstract G-88, 37th ICAAC, Toronto (1997); Anderson, E.L., Kennedy, D.J., Geldmacher, K.M., Donnelly, J., Mendelman, P; The Journal of Paediatrics, May 1996. Vo. 128, n°5, Part 1, 649-653).

Human Respiratory Syncytial Virus (RSV) is a member of the Paramyxoviridiae family of viruses and causes lower respiratory tract illness, particularly in young children and babies. Recent reports suggest that RSV is an important pathogen in adults, particularly the elderly.

RSV is an enveloped virus with a non-segmented, negative strand ribonucleic acid (RNA) genome of 15,222 nucleotides that codes for 10 messenger RNAs, each coding for a single polypeptide. Three of the ten proteins are transmembrane surface proteins: the G (attachment), F (fusion) and SH proteins. Two proteins are virion matrix proteins (M and M2), three proteins are components of the nucleocapsid (N, P and L), and two proteins are nonstructural (NS1 and NS2). Two antigenically distinct strains of RSV exist, designated strain A and B. Characterization of strains from these groups has determined that the major differences reside on the G proteins, while the F proteins are conserved.

Respiratory Syncytial virus (RSV) occurs in seasonal outbreaks, peaking during the winter in temperate climates and during the rainy season in warmer climates (DeSilva LM & Hanlon MG. Respiratory Syncytial Virus: a report of a 5-year study at a children's hospital. J Med Virol 1986;19:299-305). Wherever the area, RSV tends to have a regular and predictable pattern and other respiratory viral pathogens that occur in outbreaks are rarely present concurrently (Glezen WP & Denny FW. Epidemiology of acute lower respiratory disease in children. N. Engl. J Med 1973;288:498-505).

RSV is a major cause of serious lower respiratory tract disease in children. It is estimated that 40-50% of children hospitalized with bronchiolitis and 25% of children hospitalized with pneumonia are hospitalized as a direct result of RSV infections. Primary RSV infection usually occurs in children younger than one year of age; 95% of children have serologic evidence of past infection by two years of age and 100% of the population do so by adulthood.

In infants and young children, infection progresses from the upper to the lower respiratory tract in approximately 40% of cases and the clinical presentation is that of bronchiolitis or pneumonia. Children two to six months of age are at greatest risk of developing serious manifestations of infection with RSV (primarily respiratory failure); however, children of any age with underlying cardiac or pulmonary disease, premature infants, and infants who are immunocompromised, are at risk for serious complications as well.

Symptomatic reinfection occurs throughout life and it has become increasingly apparent that RSV is an important adult pathogen as well, especially for the elderly.

RSV infection is almost certainly underdiagnosed in adults, in part because it is considered to be an infection of children. Consequently, evidence of the virus in adults is not sought in order to explain respiratory illness. In addition, RSV is difficult to identify in nasal secretions from individuals who have some degree of partial immunity to the virus, as do the large majority of adults. Young to middle-age adults typically develop a persistent cold-like syndrome when infected with RSV. Elderly individuals may develop a prolonged respiratory syndrome which is virtually indistinguishable from influenza, with upper respiratory symptoms which may be accompanied by lower respiratory tract involvement, including pneumonia. Institutionalised elderly populations are of particular concern, because they comprise large numbers of susceptible individuals clustered together. The spread of infection through such a population, many of whom have multiple medical problems which may predispose them to a more severe course of the disease, is difficult to control.

Furthermore, reports of recent studies evaluating the impact of RSV infection as a cause of hospitalisation in adults and in community dwelling healthy elderly further point to an important role of RSV infection in severe lower respiratory tract disease in these populations (Dowell SF, Anderson LJ, & Gary Jr HE, et al. J Infect Dis 1996;174:456-462; Falsey AR, Cunningham CK, & Barker WH, et al. J Infect Dis 1995;172:389-394). Dowell identified RSV as one of the four most common pathogens causing severe lower respiratory tract disease resulting in hospitalisation of adults (Dowell SF, Anderson LJ, & Gary Jr HE, et al. J. Infect Dis 1996;174:456-462). Falsey demonstrated that serious RSV infections in elderly persons are not limited to nursing homes or outbreak situations. Rather, RSV infection is a predictable cause of serious illness among elderly patients residing in the community. Similar to hospitalisations for influenza A, those related to RSV infections were associated with substantial morbidity, as evidenced by prolonged hospital stays, high intensive care admission rates, and high ventilatory support rates (Falsey AR, Cunningham CK, & Barker WH, et al. J Infect Dis 1995;172:389-394).

These studies point to the medical and economic need for an effective vaccine which can prevent severe complications of RSV infection in infants, adults and both community dwelling healthy and institutionalised elderly.

For a recombinant subunit vaccine or a killed vaccine, the use of an adjuvant is of primary importance. Alum is the only adjuvant currently licensed for human use. However, Alum has a limited adjuvant effect on the humoral response, and is known to induce mainly a Th2 type immune response (Byars NE, Nakano G, & Welch M, et al. Vaccine 1991;9:309-318). It has been found, however, that 3 De-O-acylated monophosphoryl lipid A (3D-MPL) in combination with Alum improves the humoral response and stimulates preferentially a Th1-type immunity. The Aluminium salt with 3D-MPL adjuvant is denoted Alum/3D-MPL.

The present invention provides a vaccine composition comprising:
(a) one or more *Streptococcus pneumoniae* polysaccharides either conjugated to a protein or peptide, or non-conjugated; and
(b) an RSV antigen
in" combination with an adjuvant which is a preferential stimulator of a Th1 type response.

The trend towards combination vaccines has the advantage of reducing discomfort to the recipient, facilitating scheduling, and ensuring completion of regiment; but there is also the concomitant risk of reducing the vaccine's efficacy. It would be, therefore, advantageous to make vaccine combinations which meet the needs of a population, and which, in addition, do not interfere with each other. It would be of further advantage if the combination of the vaccines results in synergy with resulting improvement of one or both vaccines efficacy, or improved correlates of protections for one or both vaccines. This is achieved by the vaccine composition of the invention which is of great benefit for administration to children or the elderly who may be particularly at risk of *Streptococcus pneumoniae,* and/or RSV infection.

Optionally the vaccine composition of the invention additionally comprises one or more of a number of other antigens such as an *influenza* virus antigen. Currently available influenza vaccines include whole inactivated virus vaccines, split particle vaccines, and subunit vaccines. Influenza vaccines, of all kinds, are usually trivalent vaccines. They contain antigens derived from two influenza A virus strains and one influenza B strain. A standard 0.5ml dose of most of them contain 15µg of haemagglutinin component from each strain.

The procedure for determining the strains to be incorporated into an influenza vaccine is a complex process which involves collaboration between the World Health Organisation, national health authorities and vaccine manufacturers.

Pneumococcal polysaccharides can be rendered more immunogenic by chemically coupling them to protein carriers, and clinical efficacy trials are being performed to verify this concept for efficacy in preventing infant Otitis media.

There are two conjugation methods generally used for producing immunogenic polysaccharide constructs: (1) direct conjugation of carbohydrate and protein; and (2) indirect conjugation of carbohydrates and protein via a bifunctional linker or spacer reagent. Generally, both direct and indirect conjugation require chemical activation of the carbohydrate moiety prior to derivatisation. See for example US 5,651,971 and Dick & Beurret, "Glycoconjugates of Bacterial Carbohydrate Antigens," Conjugate Vaccines, J.M. Cruse & R.E. Lewis (eds), Vol. 10, 48 - 114 (1989).

The *Streptococcus pneumoniae* polysaccharide in the composition according to the invention is preferably but not necessarily in a conjugated form. Non-conjugated *S.pneumoniae* polysaccharide is not excluded.

The *Streptococcus pneumoniae* polysaccharides, if conjugated, are conjugated to either a protein or a peptide. Polysaccharide antigens have been conjugated to a number of T helper proteins. These provide T-helper epitopes. Representative proteins include Diphtheria Toxoid, Tetanus toxoid, and protein D or its lipidated derivative lipoprotein D from Heamophilius influenzae B. Other suitable protein carriers include, Diphtheria Crm 197 and the major non structural protein from influenzae, NS1 (particularly amino acid 1-81).

Protein D is a preferred component of a vaccine according to the invention in which the Streptococcus pneumoniae polysaccharides are conjugated. Fragments of protein D are also suitable. Protein D is described in EP 0 594 610. Fragments suitable for use include fragments encompassing T-helper epitopes. In particular a protein D fragment will preferably contain the N-terminal 1/3 of the protein.

Surprisingly, it has additionally been found that combining protein D-conjugated *Streptococcus pneumoniae* polysaccharide with an RSV antigen in the composition according to the invention does not affect the immune response to the protein D component of the conjugate. Protein D is a protective antigen against non-typable *Haemophilus influenzae* B (NTHi) infection.

Thus the invention further provides a vaccine comprising an RSV antigen and at least one *Streptoccocus pneumoniae* polysaccharide conjugated to protein D or a fragment thereof, optionally together with an adjuvant such as a Th1 adjuvant.

Typically the *Streptococcus pneumoniae* component in a vaccine of the present invention will comprise polysaccharide antigens (preferably conjugated), wherein the polysaccharides are derived from at least four serotypes of pneumococcus. Preferably the four serotypes include 6B, 14, 19F and 23F. More preferably, at least 7 serotypes are included in the composition, for example those derived from serotypes 4, 6B, 9V, 14, 18C, 19F, and 23F. More preferably still, at least 11 serotypes are included in the composition, for example the composition in one embodiment includes capsular polysaccharides derived from serotypes 1, 3, 4, 5, 6B, 7F, 9V, 14, 18C, 19F and 23F (preferably conjugated). In a preferred embodiment of the invention at least 13 or at least 15 or at least 17 polysaccharide antigens (preferably conjugated) are included, although further polysaccharide antigens, for example 23 valent (such as serotypes 1, 2, 3, 4, 5, 6B, 7F, 8, 9N, 9V, 10A, 11A, 12F, 14, 15B, 17F, 18C, 19A, 19F, 20, 22F, 23F and 33F), are also contemplated by the invention.

For elderly vaccination (for instance for the prevention of pneumonia) it is advantageous to include serotypes 8 and 12F (and most preferably 15 and 22 as well) to the 11 valent antigenic composition described above to form a 15 valent vaccine, whereas for infants or toddlers (where otitis media is of more concern) serotypes 6A and 19A are advantageously included to form a 13 valent vaccine.

Suitable RSV antigens for inclusion in vaccines according to the invention include inactivated RSV virus, such as a chemically inactivated RSV virus inactivated with eg formalin. The inactivated virus may be produced for example from an attenuated RSV which has been attenuated eg by passaging or by genetic manipulation, or it may be produced from a wild type RSV. A recombinant RSV may be used which has been engineered to contain an envelope antigen from a different strain of RSV eg an RSV strain A backbone with an RSV B envelope protein, optionally attenuated.

Suitable RSV antigens include, in particular antigens derived from the RSV virus, preferably human RSV envelope glycoproteins, such as the RSV F or G protein or immunogenic fragments thereof as disclosed for example in US Patent 5,149,650, or a chimeric polypeptide comprising at least one immunogenic fragment from both RSV F and G proteins, advantageously an RSV FG chimeric protein as disclosed for example in US patent 5,194,595 preferably expressed from CHO cells.

Preferably the RSV antigen is a RSV envelope glycoprotein or derivative thereof. Preferably the antigen is derived from RSV strain A. Alternatively the antigen may be derived from strain B, or there may be an antigen from each of strains A and B in the composition. The preferred antigens are selected from F glycoprotein, G glycoprotein or an FG fusion protein or immunogenic derivatives thereof.

Typically immunogenic derivatives include wherein the protein is devoid of the transmembrane domain ie F Δtm, G Δtm and F Δtm GΔtm. Preferably the signal sequence is deleted from the G protein. It is preferred that at least about 50 % or at least about 80% (contiguous sequence) of the extracellular domain of the F or G protein is present. Particular examples include 1-526 or 1-489 amino acid of the F protein, amino acid 69-298 or alternative positions 97-279 of the G protein, and F₁₋₅₂₆G₆₉₋₂₉₈ fusion protein. An alternative fusion comprises 1-489 amino acid from F followed by 97-279 of G protein.

The RSV / *Streptococcus pneumoniae* vaccine composition according to the invention comprises an adjuvant which is a preferential inducer of a Th1 type of response to aid the cell mediated branch of the immune system.

An immune response is generated to an antigen through the interaction of the antigen with the cells of the immune system. The resultant immune response may be broadly distinguished into two extreme catagories, being humoral or cell mediated immune responses (traditionally characterised by antibody and cellular effector mechanisms of protection respectively). These categories of response have been termed Th1-type responses (cell-mediated response), and Th2-type immune responses (humoral response).

Extreme Th1-type immune responses may be characterised by the generation of antigen specific, haplotype restricted cytotoxic T lymphocytes, and natural killer cell responses. In mice Th1-type responses are often characterised by the generation of antibodies of the IgG2a subtype, whilst in the human these correspond to IgG1 type antibodies. Th2-type immune responses are characterised by the generation of a broad range of immunoglobulin isotypes including in mice IgGI, IgA, and IgM.

It can be considered that the driving force behind the development of these two types of immune responses are cytokines, a number of identified protein messengers which serve to help the cells of the immune system and steer the eventual immune response to either a Th1 or Th2 response. Thus high levels of Th1-type cytokines tend to favour the induction of cell mediated immune responses to the given antigen, whilst high levels of Th2-type cytokines tend to favour the induction of humoral immune responses to the antigen.

It is important to remember that the distinction of Th1 and Th2-type immune responses is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman *(*Mosmann, T.R. and Coffman, R.L. (1989) TH1 and TH2 cells: different patterns of lymphokine secretion lead to different functional properties. Annual Review of Immunology, 7, p145-173*).* Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2- type responses are associated with the secretion of IL-4, IL-5, IL-6, IL-10 and tumour necrosis factor-β(TNF-β).

It is known that certain vaccine adjuvants are particularly suited to the stimulation of either Th1 or Th2 - type cytokine responses. Traditionally the best indicators of the Th1 :Th2 balance of the immune response after a vaccination or infection includes direct measurement of the production of Th1 or Th2 cytokines by T lymphocytes *in vitro* after restimulation with antigen, and/or the measurement of the IgG1 :IgG2a ratio of antigen specific antibody responses.

Thus, a Th1-type adjuvant is one which stimulates isolated T-cell populations to produce high levels of Th1-type cytokines when re-stimulated with antigen *in vitro,* and induces antigen specific immunoglobulin responses associated with Th1-type isotype.
Suitable adjuvant systems which promote a predominantly Th1 response include Monophosphoryl lipid A or a derivative thereof, particularly 3-de-O-acylated monophosphoryl lipid A, and a combination of monophosphoryl lipid A, preferably 3-de-O-acylated monophosphoryl lipid A (3D-MPL) together with an aluminium salt. Further suitable adjuvant systems are described below.

Such adjuvants are described for example in International Patent Application No. WO 94/00153 and WO 95/17209.

(3D-MPL is described in GB 2220211 (Ribi). Chemically it is a mixture of 3-de-O-acylated monophosphoryl lipid A with 4, 5 or 6 acylated chains and is manufactured by Ribi Immunochem Montana. A preferred form of 3 De-O-acylated monophosphoryl lipid A is disclosed in European Patent EP 0 689 454 B.

Preferably, the size of the particles of 3D-MPL are small enough to be sterile filtered through a 0.22micron membrane (as described in European Patent EP 0 689 454).

3D-MPL will normally be present in the range of 10µg - 100µg preferably 25-50µg per dose wherein the antigen will typically be present in a range 2-50µg per dose.

Another preferred adjuvant comprises QS21, an Hplc purified non-toxic fraction of a saponin derived from the bark of Quillaja Saponaria Molina. Optionally this may be admixed with 3-de-O-acylated monophosphoryl lipid A (3D-MPL), optionally together with a carrier.

A method of producing QS21 is disclosed (as QS21) in US patent No. 5,057,540.

Non-reactogenic adjuvant formulations containing QS21 are described in WO 96/33739. Such formulations comprising QS21 and cholesterol have been shown to be successful Th1 stimulating adjuvants when formulated together with an antigen. Thus vaccine compositions which form part of the present invention may include a combination of QS21 and cholesterol.

Further adjuvants which are preferential stimulators of Th1 response include immunomodulatory oligonucleotides, for example unmethylated CpG sequences as disclosed in WO 96/02555.

Combinations of different Th1 stimulating adjuvants, such as those mentioned hereinabove, are also contemplated as providing an adjuvant which is a preferential stimulator of Th1 type response. For example, QS21 (preferably with cholesterol also) can be formulated together with 3D-MPL. The ratio of QS21 : 3D-MPL will typically be in the order of 1 : 10 to 10 : 1; preferably 1:5 to 5 : 1 and often substantially 1 : 1. The preferred range for optimal synergy is 2.5 : 1 to 1 : 1 3D MPL: QS21.

Preferably a carrier is also present in the vaccine composition according to the invention. The carrier may be an oil in water emulsion, or an aluminium salt.

A preferred oil-in-water emulsion comprises a metabolisable oil, such as squalene, alpha tocopherol and tween 80. Additionally the oil in water emulsion may contain span 85 and/or lecithin and/or tricaprylin.

In a preferred aspect aluminium hydroxide (alum) or aluminium phosphate will be added to the composition of the invention to enhance immunogenicity.

In a particularly preferred aspect the antigens in the vaccine composition according to the invention are combined with 3D-MPL and alum.

Typically for human administration QS21 and 3D-MPL will be present in a vaccine in the range of 1 µg - 200 µ g, such as 10-100 µ g, preferably 10 µ g - 50 µ g per dose. Typically the oil in water will comprise from 2 to 10% squalene, from 2 to 10% alpha tocopherol and from 0.3 to 3% tween 80. Preferably the ratio of squalene: alpha tocopherol is equal or less than 1 as this provides amore stable emulsion. Span 85 may also be present at a level of 1%. In some cases it may be advantageous that the vaccines of the present invention will further contain a stabiliser.

Non-toxic oil in water emulsions preferably contain a non-toxic oil, e.g. squalane or squalene, an emulsifier, e.g. Tween 80, in an aqueous carrier. The aqueous carrier may be, for example, phosphate buffered saline.

A particularly potent adjuvant formulation involving QS21, 3D-MPL and tocopherol in an oil in water emulsion is described in WO 95/17210.

The vaccine of the present invention will contain an immunoprotective quantity of the antigens and may be prepared by conventional techniques.

Vaccine preparation is generally described in Pharmaceutical Biotechnology, Vol.61 Vaccine Design - the subunit and adjuvant approach, edited by Powell and Newman, Plenum Press, 1995; New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of protein in each vaccine dose is selected as an amount which induces an immunoprotective response without significant, adverse side effects in typical vaccinees. Such amount will vary depending upon which specific immunogen is employed. Generally, it is expected that each dose will comprise 1-1000 mg of protein, preferably 2-100 mg, most preferably 4-40 mg. An optimal amount for a particular vaccine can be ascertained by standard studies involving observation of antibody titres and other responses in subjects. Following an initial vaccination, subjects may receive a boost in about 4 weeks. For infants in particular, three doses may be preferred.

In addition to vaccination of persons susceptible to *Streptococcus pneumoniae* or RSV infections, the pharmaceutical compositions of the present invention may be used in the manufacture of a vaccine to treat, immunotherapeutically, patients suffering from the said infections.

In a further aspect of the present invention there is provided a method of manufacture of a vaccine formulation as herein described, wherein the method comprises mixing a *Streptococcous pneumoniae* antigen and an RSV antigen with a Th-1 inducing adjuvant, for example 3D-MPL and, preferably, a carrier, for example alum.

If desired, other antigens may be added, in any convenient order, to provide multivalent vaccine compositions as described herein.

The invention further provides a method of producing a vaccine composition which comprises mixing a *Streptococcus pneumoniae* polysaccharide conjugated to protein D with an RSV antigen and a pharmaceutically acceptable excipient.

In the examples that follow, evaluation of the effect of combining an RSV vaccine candidate, FG with the commercially available 23-valent pneumococcal vaccine (Pneumune from Wyeth-Lederle) in a mouse model was carried out. This model tested a combined immunisation where the vaccines are physically mixed and injected in the same site. The results of this study demonstrate a mutual synergy, resulting in improved responses for certain immunological markers for both vaccines.

The examples further demonstrate a combined vaccine with an RSV vaccine candidate and an 11-valent conjugated pneumococcal vaccine, in a mouse model. This study demonstrates that for most immunological markers, there is no interference or only minor interference between the antigens, but in fact for some immunological markers there is an improved response and therefore a synergistic effect. Notably, there is no reduction in the immune response to the protein D component of the conjugated pneumococcal polysaccharide.

### EXAMPLES

### Example 1 - Evaluation of combined RSV + 23-valent pneumococcal vaccine

### 1. Formulation process:

H₂O-diluted FG antigen (2µg:1/10 HD) was adsorbed for 15 min on 50 µg of Al(OH)₃. When used, 5µg of 3D-MPL were added to the preparation as a suspension of 100 nm particles and incubated for 30 min. Formulations were then buffered with 10-fold concentrated PBS pH 7.4. When used, the 23 valent pneumococcal vaccine (11.5µg:1/50 HD) was added 15 min. after the addition of the buffer solution. For groups without FG the same formulation sequence was followed so that the 3D-MPL was first adsorbed on Al(OH)₃ before adding the 23 valent pneumococcal vaccine. Fifteen minutes after the addition of the concentrated buffer or the 23 valent pneumococcal vaccine phenoxyethanol (5mg/ml) was added to the formulations as preservative.

All incubations were carried out at room temperature with agitation. The formulations were prepared simultaneously for the 2 injections with a 7-day maturation of the finalised formulations before the first injection.

### Composition of formulation constituents:

| **COMPONENT** | **BATCH NUMBER** | **CONCENTRATION (µg/ml)** | **BUFFER** |
|---|---|---|---|
| FG | 54/023 | 265 | 10 PO4, 150NaCl pH6.8 |
| Al (OH)₃ | 96A0089 | 10380 | H₂O |
| 23-Valent | Pneumune | 1150 | saline |
| 3D-MPL | 109 | 964 | H₂O |

### 2. Immunization protocol:

11 groups of 10 mice were immunised by different routes (50 µl) at days 0 and 28 with various formulations (see ***Table 1***). Group 7 and 8 were immunised with live RSV by the intra-nasal route (60µl). Sera were obtained at days 28 (28 d Post I) and 42 (14 d Post II). On day 42, spleen and node cells were taken from 5 mice of groups 4, 5, 6, 7, 8, 9, as well as from 5 naive Balb/c mice (group 1, not immunised).

### 3. Humoral response.

### 3.1. Anti-FG antibodies:

All humoral results were performed for 10 mice/group (individual response for the anti-FG titers and pooled sera for the isotype profile) and cellular results were presented for 5 mice/group.

Individual sera were obtained 28 days after the first Immunization and 14 days after the second immunisation and were tested for the presence of FG specific Ig antibodies and their isotype (IgG2a, IgG1) distribution.

The assay protocol was as follows: coating overnight at 4°C with 50 µl of purified FG 54/023 (1µg/ml) per well, saturation 1h at 37°C, incubation with sera 1h30 at 37°C, incubation with anti-mouse Ig biotin 1/1500 (or IgG1, IgG2a biotin 1/1000) 1h30 at 37°C, incubation with strepta-peroxydase 1/2500 30 min at 37°C, incubation with OPDA Sigma 15 min at RT, stop with H₂SO₄ 2N.

OD were monitored at 490 nm and the titers determined by Softmaxpro (4 parameters equation) referring to a standard curve and expressed in EU/ml.

Individual sera obtained 14d Post II were tested for the presence of neutralising antibodies using the following protocol: 50 µl of serial two-fold dilutions of sera (first dilution 1/25) were incubated for 1 hour at 37°C with 50 µl of a mixture containing 300 pfu of RSV-A/Long (Lot 14) and guinea pig complement (1/25 dilution) in a 96 well plate in duplicate. 100 µl of a HEp-2 cell suspension at 10⁵ cells/ml were then added to each well and the plates were incubated for 4 days at 37°C in the presence of 5% CO₂.

The supernatants were then aspirated, and after addition of a 100 µl of a WST-1 preparation (dilution 1/12.5) the plates were further incubated for 24H at 37°C in the presence of 5% CO₂. The OD were monitored at 595 nm and the titers determined by linear regression (y=a.logx + b): titer = serum dilution giving 50% reduction of the maximal OD observed for the uninfected cells.

Controls in test included a pool of randomly chosen human sera (Human pool) and Sandoglobuline (lot 069, generic human IgG produced by Sandoz).

### 3.2. Anti-Pneumococcal Polysaccharide IgG:

Murine IgG to pneumococcal polysaccharides types 6B, 14, 19F and 23F was measured by ELISA in a method adapted from the CDC protocol. This protocol includes the addition of soluble cell wall polysaccharide (CPS) to the sera to inhibit the measurement of CPS antibodies. CPS is a phosphoryl choline containing teichoic acid common to all pneumococci. It is present under the capsule, and antibodies to it are only weakly protective. Since CPS is linked to the capsular polysaccharide, there is usually 0.5 to 1% CPS contaminating the purified capsular polysaccharide used to coat the ELISA plates. Thus, measurement of the CPS antibodies can confound the interpretation ELISA results with respect to the capsular polysaccharide.

The ELISA was performed with polysaccharides coated at 20, 5, 10 and 20 µg/ml in carbonate buffer for types 6B, 14, 19F and 23F respectively. Sera was pre-mixed with the equivalent of 500 µg/ml CPS in undiluted sera, and incubated for 30 minutes before addition to the ELISA plate. Murine IgG was detected with Jackson ImmunoLab goat anti-murine IgG (H+L) peroxidase at 1:2000 dilution. The titration curves were referenced to polysaccharide specific murine monoclonal antibodies of known concentration for each serotype using logistic log comparison by SoftMax Pro. The monoclonals used were HASP4, PS 14/4, PS-19/5 and PS23/22 for types 6B, 14, 19F and 23F respectively. Due to the limited quantity of sera available, pooled sera was tested, thus statistical analysis is not available.

### 4. Cellular response

Spleen and lymph node cells were isolated 14d Post II from groups 4-9 and from naïve mice (Group 1) for use as a negative control for the FG-specific cellular response analysis. Samples were analysed for both FG-specific lymphoproliferation and cytokine (IFN-γ + IL-5) secretion.

Proliferation was evaluated after a 96h incubation of 4x10⁵ cells/well of 96 well plates with 200 µl of media containing 10 to 0.03 µg/ml ofFG (3-fold dilutions). Upon ³H-thymidine incorporation, the FG specific proliferation was measured following our standard protocol. Cytokine induction was evaluated after 96 h incubation of 2.5x10⁶ cells per well of 24 well with 1 ml of media containing 10µg to 0.01 µg of FG (10-fold dilutions). Supernatants were then harvested to determine the quantity of IFN-γ and IL-5 induced by ELISA following our standard protocol.

### 1. GROUPS.

10 groups received two immunisations of various formulations containing either the 23-valent vaccine or FG or a combination of both formulated with Aluminium hydroxide and 3D-MPL or Aluminium hydroxide. Group 1 constitutes the control for the CMI studies. Groups 2 and 9 will allow the evaluation of the immunogenicity of the 23 Valent pneumoccocal vaccine upon intraperitoneal immunization which was used in the literature for the evaluation of 23 Valent pneumoccocal vaccine, and upon IM immunization, Groups 3 and 10 allow a parallel analysis to groups 2 and 9 except that the 23 Valent pneumoccocal vaccine is formulated with Alum/3D-MPL. Groups 2 and 3 also constitute controls for the impact of Alum/3D-MPL on the 23 Valent pneumoccocal vaccine when it is not combined to FG Alum/3D-MPL. Group 4 will allow the evaluation of the immune response induced upon IM Immunization of the combination of the 23 Valent pneumoccocal vaccine and FG Alum/3D-MPL. Groups 5 and 6 constitute a control for the evaluation of the impact of Alum/3D-MPL on FG when it is not combined with the 23 Valent pneumoccocal vaccine. The RSV live immunisations was a control for the immune response induced upon natural RSV IN infection (Group 7). Finally, group 9 is a control for the impact of Alum/3D-MPL alone.

**TABLE 1**

| Groups | Antigen | Adjuvant | Route |
|---|---|---|---|
| 1 | none | none | |
| 2 | 23 Valent Pneumune( 11. 5 µg) | none | IM |
| 3 | 23 Valent Pneumune (11. 5 µg) | Alum/3D-MPL | IM |
| 4 | 23 Valent Pneumune (11.5µg) / FG (2µg) | Alum/3D-MPL | IM |
| 5 | FG (2µg) | Alum/3D-MPL | IM |
| 6 | FG (2µg) | Alum | IM |
| 7 | RSV Live (Lot 14: 10⁵ PFU) | none | IN |
| 8 | none | Alum/3D-MPL | IM |
| 9 | 23 Valent Pneumune (11.5 µg) | none | IP |
| 10 | 23 Valent Pneumune (11.5µg) | Alum/3D-MPL | IP |

### 2. HUMORAL RESPONSE.

### 2.1. Anti-FG antibodies

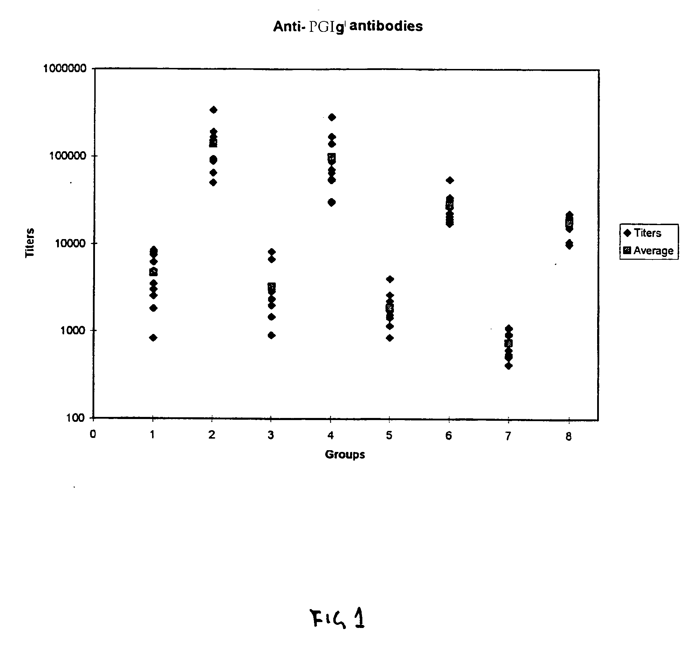

The analysis of specific anti-FG antibodies at 28 d Post I and 14 d Post II shows the induction of similar antibody responses upon Immunization with FG Alum/3D-MPL / 23-Valent (Group 4) or FG Alum/3D-MPL alone (Group 5) with slightly higher titers observed for FG Alum/3D-MPL / 23-Valent **(*****Figure 1*****)*.*** The Immunization with FG Alum (Group 6) induces expectedly lower antibody titers at both time points. Statistical analysis shows that there is indeed a significant difference between the anti-FG Ig titers induced by Groups 4 -6 at 28 d Post I and 14 d Post II.

### RESULTS

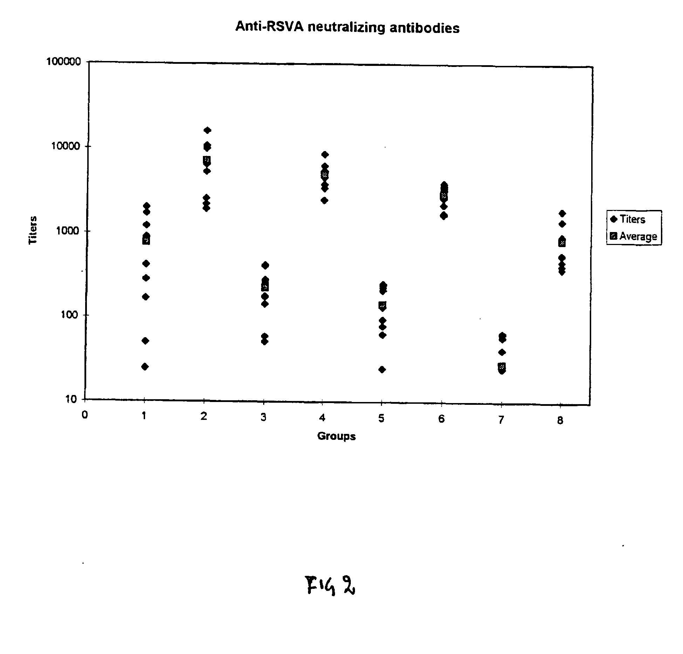

The analysis of anti-RSVA neutralizing antibodies *(**Figure 2**)* presents a parallel profile as the one observed for the anti-FG Ig responses.

Based on the above results, the anti-FG / anti RSV neutralizing antibody ratio (28d PostII) was calculated and showed that the ratio of FG Alum/3D-MPL / 23-Valent, FG Alum/3D-MPL and FG Alum compares to the ratio induced by the RSV IN group *(**Table 2**).*

**TABLE 2**

| **Vaccine Candidate** | **Group (#)** | **Antibody Titers (GMT)** | | | | | |
|---|---|---|---|---|---|---|---|
| | | **28 d Post I** | | | **14 d Post II** | | |
| | | **Anti FG ELISA Ig** | **RSV-A Neutra** | **ELISA/Neutra Ratio** | **Anti-FG ELISA 1g** | **RSV-A Neutra** | **ELISA/Neutra ratio** |
| **FG SB AS4 / 23 valent** | **4** | **3836** | **392** | **10** | **121707** | **5809** | **21** |
| **FG SB AS4** | **5** | **2713** | **187** | **15** | **78079** | **4664** | **17** |
| **FG Alum** | **6** | **1721** | **117** | **15** | **26163** | **2771** | **9** |
| **Live RSV** | **7** | **703** | **35** | **20** | **17119** | **726** | **24** |

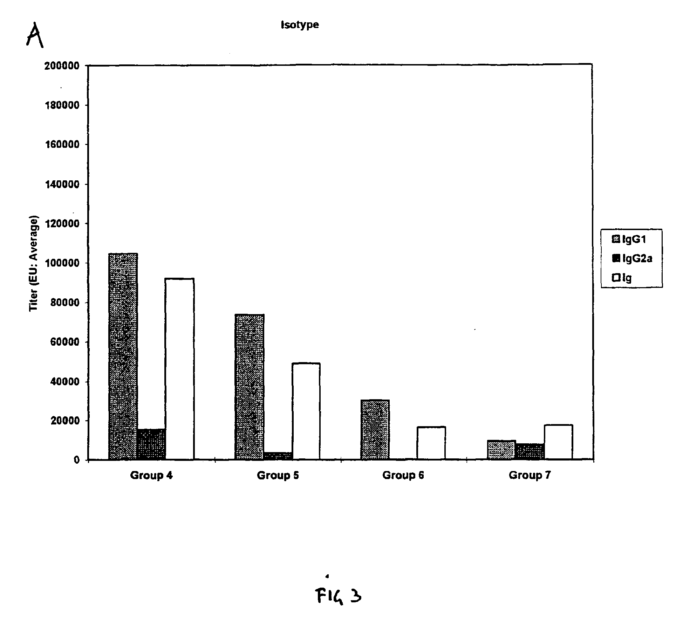

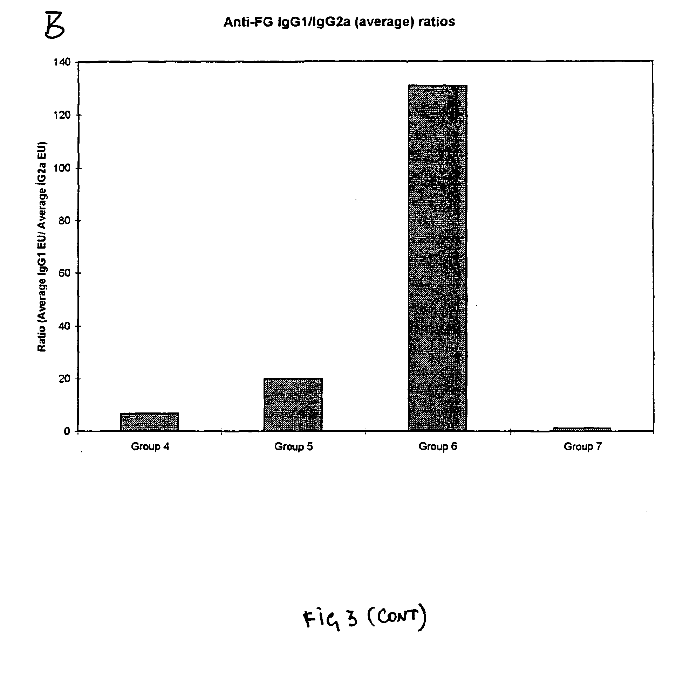

An in depth statistical analysis on individual anti-FG Ig, IgG1 and IgG2a isotype titers showed that the addition of the 23-valent vaccine to FG Alum/3D-MPL maintains the Ig and IgG1 responses and increases significantly the IgG2a responses ***(******Figure 3A******)**.* In addition, the analysis showed that the IgG1/IgG2a ratio and the IgG1 titer of FG Alum/3D-MPL / 23 valent and FG Alum/3D-MPL were similar although significantly different from FG Alum ***(******Figure 3B******)**.* The three formulations had significant differences in their IgG2a titers.

### 2.2. Anti-Pneumococcal Polysaccharide IgG

While mice and other rodents may produce IgM against polysaccharide immunogens, they do not normally produce IgG against polysaccharides. This is because their immune system lacks the signals to induce isotype switching to a T-independent antigen such as a polysaccharide. Thus, the measurement of anti-polysaccharide IgG in mice is a sensitive test for the induction of an improved T-independent immune response.

The concentrations of IgG induced against four of the 23 polysaccharides are presented in ***Table 3***. As we had noted in other experiments, no IgG was produced against polysaccharide types 6B and 23F. However, there was measurable IgG produced against types 14 and 19F.

Groups that did not contain 23-Valent vaccine did show any detectable IgG to polysaccharides 14 and 19F, confirming the specificity of the measurements.

A comparison of the route of immunisation reveals that when the 23-valent vaccine is adjuvanted with Alum/3D-MPL, the intra-muscular route appears to be better than inter-peritoneal for type 19F, whereas the reverse is true for type 14. With plain 23-Valent, there does not seem to be a significant difference.

23Valent + Alum/3D-MPL induces greater IgG for types 14 and 19F, in both IM and IP immunisation routes when compared to 23-Valent alone. When combined with FG, however, there is a reduction in the IgG response. Nevertheless, the IgG response to 19F in 23-Valent+FG/Alum/3D-MPL is still greater than that induced by 23-Valent alone (compare groups 3 and 4), and the response to type 14 is improved.

**TABLE 3**

| **Combination RSV Pneumococcal Vaccine Murine IgG (µg/ml)** | | | | | | | | | |
|---|---|---|---|---|---|---|---|---|---|
| | **Group 1** | **Group 2** | **Group 3** | **Group 4** | **Group 5** | **Group 6** | **Group 8** | **Group 9** | **Group 10** |
| | **/** | **IM 23 Valent** | **IM 23 Valent MPL Al(OH)3** | **IM 23 Valent/FG MPL Al(OH)3** | **IM FG MPL Al(OH)3** | **IM FG Al(OH)3** | **IM / MPL Al(OH)3** | **IP 23 Valent** | **IP 23 Valent MPL Al(OH)3** |
| **Anti PS6B** | **<1.2** | **<1.2** | **<1.2** | **<1.2** | **<1.2** | **<1.2** | **<1.2** | **<1.2** | **<1.2** |
| **Anti PS14** | **<0.04** | **0.1** | **0.6** | **0.2** | **<0.04** | **<0.04** | **<0.04** | **0.2** | **1.2** |
| **Anti PS19F** | **<1.2** | **1.7** | **3.8** | **2.3** | **<1.2** | **<1.2** | **<1.2** | **1.6** | **1.6** |
| **Anti PS23F** | **<1** | **<1** | **<1** | **<1** | **<1** | **<1** | **<1** | **<1** | **<1** |

### 3. CELLULAR RESPONSE.

The induced FG-specific lymphoproliferation did not show any difference between FG Alum/3D-MPL +/- 23 Valent and FG Alum, both in spleen cells and in lymph node cells.

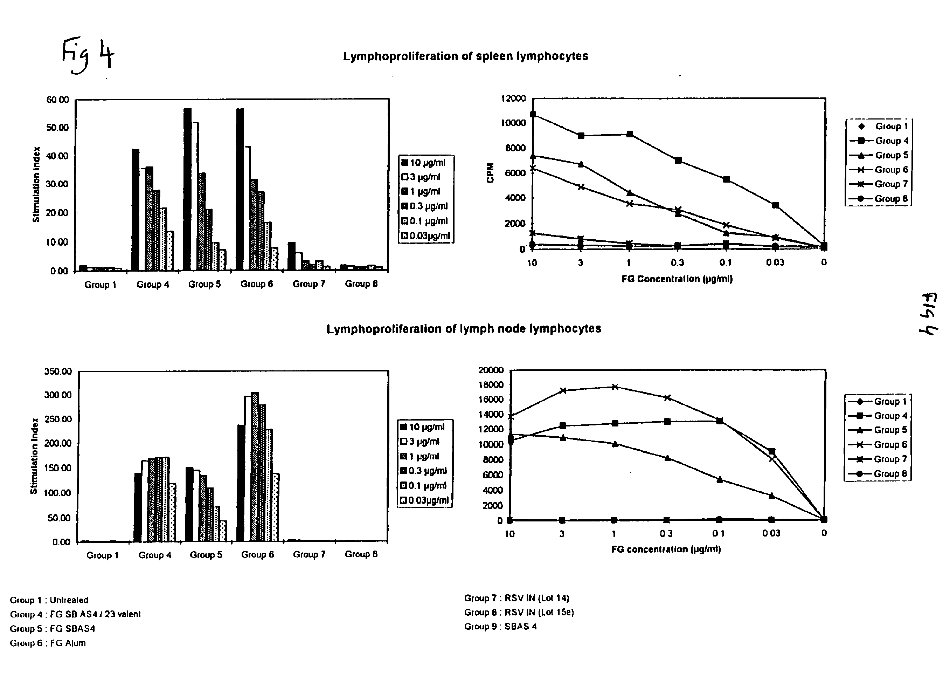

The analysis of the production of IL-5 and IFN-γ suggests that the mixture of the 23 valent vaccine with FG Alum/3D-MPL does not hamper the production of IFN-γ but could increase somewhat the production of IL-5 **(*****Figure 4*****)*.*** This observation is confirmed by a 2 to 8 fold difference in the IFN-γ / IL-5 ratio observed at two doses of FG (10 and 1 µg FG/ml) used for *in vitro* restimulation. However, the FG Alum/3D-MPL /23-valent vaccine still induces a much higher IFN-γ / IL-5 ratio than FG Alum.

### CONCLUSIONS

The analysis of the induced anti-FG Ig specific and anti RSVA neutralizing antibody titers shows that the combination of FG Alum/3D-MPL with the 23 valent pneumococcal vaccine does not hamper the induction of the response observed with FG Alum/3D-MPL alone. The quality of the response measured by the anti-FG/anti-RSVA neutralizing antibody ratio also remains unchanged and close to the ratio observed upon natural infection with RSV true the IN route.

Analysis of the induced FG specific cell mediated response suggest that the addition of the 23 valent vaccine to FG Alum/3D-MPL does not affect the induction of lymphoproliferation in both spleen cells and lymph node cells. Furthermore, the induction of a Th1 type response typically observed with FG Alum/3D-MPL is not hampered by the addition of the 23 valent as measured by in vitro cytokine production of FG specific spleen and lymphnode cells and appears to be enhanced when analysing the FG specific isotype antibody distribution. Indeed, the production of IFN-γ, a marker of a Th1 response, remains unchanged upon addition of the 23 valent vaccine to FG Alum/3D-MPL while the production of Il-5, marker of a Th2 response is only slightly increased. Interestingly, the addition of the 23 valent vaccine to FG Alum/3D-MPL significantly increases the production of IgG2a antibodies, marker of a Th1 response, while it is not affecting the IgG1 (marker of a Th2 response) nor the IgG1/IgG2a response.

Thus the combination of FG Alum/3D-MPL and 23-valent vaccine does not affect the response observed with FG Alum/3D-MPL and therefore the advantages linked to the FG Alum/3D-MPL formulation versus FG Alum i.e., induction of high primary and secondary neutralizing antibody responses and a Th1 response as measured by the presence of IgG2a antibodies and the induction by high levels of IFN-γ are maintained.

Combination of 23Valent Pneumune with FG Alum/3D-MPL results in increased IgG production in 2 of 4 polysaccharides tested, but most dramatically for type 19F. 23-Valent alone with Alum/3D-MPL gave the highest IgG concentrations.

In conclusion, there is a favourable combination of 23-Valent pneumococcal vaccine with RSV FG Alum/3D-MPL in which there is a synergistic effect for both vaccines in that both vaccines show improvements in certain immunological tests, and no inhibition in any tests.

### EXAMPLE 2 - Evaluation of a combined RSV + 11-valent conjugated pneumococcal vaccine

The combination vaccine was evaluated with either 3D-MPL + alum, 3D-MPL + QS21 in cholesterol containing liposomes, or CpG containing oligonucleotides (with or without alum).

The impact of the combination vaccine on the IgG immune response to protein D (PD) carrier was also assessed.

PD is a protective antigen against non typable *Haemophilus influenzae* B (NTHi) infection. Therefore, combining PD-conjugated pneumococcal PS and FG could confer an effective protection against three pathogens: *S. pneumoniae,* RSV and non typable *Haemophilus influenzae* B. A Th1 type immunity is probably involved in the protection against NTHi. Therefore, anti-PD IgG2a antibodies, which are a good indicator of a Th1 response, were also tested.

The 11-valent candidate vaccine used in this example includes the capsular polysaccharides serotypes 1, 3, 4, 5, 6B, 7F, 9V, 14, 18C, 19F and 23F which were made essentially as described in EP 72513. Each polysaccharide is activated and derivatised using CDAP chemistry (WO 95/08348) and conjugated to the protein carrier. All the polysaccharides are conjugated in their native form, except for the serotype 3 (which was size-reduced to decrease its viscosity).

The protein carrier was recombinant protein D (PD) from Non typeable *Haemophilus influenzae,* expressed in *E*. *coli.*

### 1. Formulation process:

Formulations were prepared 12 days before the first injection.

### 1.1. QS21, 3D-MPL based formulations

When needed, antigens (FG (2µg) or/and non-adsorbed PS conjugates (0.5µg/valence)) were diluted in a mix of 10-fold concentrated PBS pH 7.4 and H₂O. QS21 (5 µg) was added as a mix with liposomes containing MPL in a weight ratio QS21/Cholesterol of 1/5 (referred to as DQ/3D-MPLin). Thirty minutes later 1 µg/ml of thiomersal was added as preservative.

### 1.2. CpG or 3D-MPL based formulations

When needed, Al(OH)₃ was diluted in H₂O before addition of the antigens (FG (2µg) or/and non-adsorbed PS conjugates (0.5µg/valence)). After an adsorption of 30 min, MPL or CpG was added. Thirty minutes later the formulations were buffered with 10 fold concentrated PO₄-NaCl pH 6.8. Thiomersal at 1 mg/ml or phenoxy at 5mg/ml was then added as preservative.

All incubations were carried out at room temperature with agitation. The formulations were prepared simultaneously for the 2 injections with a 7-day maturation of the finalized formulations before the first injection.

### 1.3. Composition of formulation constituents:

| **COMPONENT** | **AG OR IMST CONC (µG/ML)** | **AL+++ CONC (µG/ML)** | **BUFFER** |
|---|---|---|---|
| FG | 484 | | PO4/NaCl 10/150mM pH 6.8 |
| Clinical undecaval non ads | | | |
| PS1 | 144 | | NaCl 150mM pH6.1 |
| PS3 | 149 | | NaCl 150mM pH6.1 |
| PS4 | 125 | | NaCl 150mM pH6.1 |
| PS5 | 135 | | NaCl 150mM pH6.1 |
| PS6b | 206 | | NaCl 150mM pH6.1 |
| PS7 | 168 | | NaCl 150mM pH6.1 |
| PS9 | 175 | | NaCl 150mM pH6.1 |
| PS14 | 180 | | NaCl150mM pH6.1 |
| PS18 | 127 | | NaCl150mM pH6.1 |
| PS 19 | 140 | | NaCl 150mM pH6.1 |
| PS23 | 158 | | NaCl 150mM pH6.1 |
| QS21 | 2000 | | H₂O |
| MPL | 1019 | | H₂O |
| CpG | 5000 | | H₂O |
| SUV | DOPC 40000 Chol 10000 MPL2000 | | PBS pH7.4 |
| AlPO₄ | | 1000 | NaCl 150mM pH6. 1 |
| Al(OH)₃ | | 10380 | H₂O |

### 2. Immunization protocol:

14 groups of 10 mice were immunized by the IM route (50 µl) at days 0 and 28 with various formulations (see *Results section).* Sera were obtained at day 42 (14 d Post II). On day 42, spleen cells were taken from 5 mice of groups immunized with FG antigen as well as from 5 naïve Balb/c mice.

### 3. Humoral response.

### 3.1. Anti-FG antibodies:

All humoral results were performed for 10 mice/group (individual response for the anti-FG Ig, IgG1 and IgG2a and neutralization titers) and cellular results were presented for 5 mice/group on pool.

Individual sera were obtained 14/15 days after the second immunization and were tested for the presence of FG specific Ig antibodies and their isotype (IgG2a, IgG1) distribution. The assay protocol was as follows: coating overnight at 4°C with 50 µl of purified FG DFGP107 (1µg/ml) per well, saturation 1h at 37°C, incubation with sera 1h30 at 37°C, incubation with anti-mouse Ig biotin 1/1500 (or IgG1, IgG2a biotin 1/1000) 1h30 at 37°C, incubation with strepta-peroxydase 1/1500 30 min at 37°C, incubation with OPDA Sigma 20 min at RT, stop with H₂SO₄ 2N.

OD were monitored at 490 nm and the titers determined by Softmaxpro (4 parameters equation) referring to a standard curve and expressed in EU/ml.

Individual sera obtained on 14d Post II were tested for the presence of neutralizing antibodies using the following protocol: 50 µl of serial two-fold dilutions of sera (first dilution 1/25) were incubated for 1 hour at 37°C with 50 µl of a mixture containing 3000 pfu of RSV-A/Long (Lot 14) and guinea pig complement (1/25 dilution) in a 96 well plate in duplicate. 100 µl of a HEp-2 cell suspension at 10⁵ cells/ml were then added to each well and the plates were incubated for 4 days at 37°C in the presence of 5% CO₂.

The supernatants were then aspirated, and after addition of a 100 µl of a WST-1 preparation (dilution 1/15) the plates were further incubated for 24H at 37°C in the presence of 5% CO₂. The OD were monitored at 490 nm and the titers determined by linear regression (y=a.logx + b): titer = serum dilution giving 50% reduction of the maximal OD observed for the uninfected cells.

### 3.2. Anti-Pneumococcal Polysaccharide IgG:

Murine IgG to pneumococcal polysaccharide types 3, 6B, 7F, 14, 19F and 23F was measured by ELISA in a method adapted from the CDC protocol. This protocol includes the addition of soluble cell wall polysaccharide (CPS) to the sera to inhibit the measurement of CPS antibodies. CPS is a phosphoryl-choline containing teichoic acid common to all pneumococci. It is present under the capsule, and antibodies to it are only weakly protective. Since CPS is linked to the capsular polysaccharide, there is usually 0.5 to 1 % CPS contaminating the purified capsular polysaccharide used to coat the ELISA plates. Thus, measurement of the CPS antibodies can confound the interpretation ELISA results with respect to the capsular polysaccharide.

The ELISA was performed with polysaccharides coated at 20, 5, 5, 20 and 20 µg/ml in PBS buffer for types 6B, 7F, 14, 19F and 23F respectively. Sera was pre-mixed with the equivalent of 500 µg/ml CPS in undiluted sera, and incubated for 30 minutes before addition to the ELISA plate. Murine IgG was detected with Jackson ImmunoLab goat anti-murine IgG (H+L) peroxidase at 1:5000 dilution. The titration curves were referenced to polysaccharide specific murine monoclonal antibodies of known concentration for each serotype using logistic log comparison by SoftMax Pro. The monoclonal antibodies used were HASP4, PS7/19, PS14/4, PS19/5 and PS23/22 for types 6B, 7F, 14, 19F and 23F respectively.

For serotype 3, a similar ELISA was done except that immunoplates were first coated with methylated human serum albumin (1 µg/ml in PBS, 2 hours, 37°C) in order to improve the PS3 coating (2.5 µg/ml in PBS, overnight, 4°C). Monoclonal antibody PS3/6 was used as standard reference.

### 3.3. ELISA dosage of anti-PD serum IgG

Maxisorp Nunc immunoplates were coated at 37°C for 2 hours with 100 µl/well of 2 µg/ml PD antigen diluted in PBS (in rows B to H of plate), or with 100 µl of 1 µg/ml purified goat anti-mouse Ig (Jackson), in PBS (row A). Then, serial 2-fold dilutions (in PBS Tween20 0.05% buffer, 100 µl per well) of pure mouse IgG (Jackson) added as a standard curve (starting at 1 µg/ml and put in row A) and serum samples (starting at a 1/20 dilution and put in rows B to H) were incubated overnight at 4°C. Then, Peroxydase-conjugated goat anti-mouse IgG (Jackson) diluted 1/5000 in PBS Tween20 0.05% buffer were incubated (100 µl/well) for 30 minutes, at 20°C, under agitation. After several washings, plates were incubated for 15 min at room temperature in the darkness with 100 µl/well of revelation buffer (OPDA 0.4 mg/ml (Sigma) and H₂O₂ 0.05 % in 100mM pH 4.5 citrate buffer). Revelation was stopped by adding 50 µl/well HC11N. Optical densities were read at 490 and 620 nm by using Emax immunoreader (Molecular Devices). Antibody titre were calculated by the 4 parameter mathematical method using SoftMaxPro software.

### 3.4. ELISA dosage of anti-PD serum IgG1, 2a and 2b

Maxisorp Nunc immunoplates were coated overnight at 4°C with 50 µl/well of 2 µg/ml PD antigen diluted in PBS (in rows B to H of plate), or with 50 µl of µg/ml purified goat anti-mouse Ig (Boerhinger), in PBS (row A). After plate saturation with PBS Tween20 0.05% BSA 1% buffer, serial 2-fold dilutions (in saturation buffer, 50 µl per well) of pure mouse IgG1, IgG2a or IgG2b monoclonal antibodies (Sigma) added as a standard curve (starting at 200 ng/ml and put in row A) and serum samples (starting at a 1/20 dilution in saturation buffer and put in rows B to H) were incubated for 1 hour at 37°C under agitation. After several washings with NaCl 0.9% Tween-20 0.05% buffer, biotinylated anti-mouse IgG1 or anti-mouse IgG2a or anti-mouse IgG2b goat IgG (Amersham) diluted 1/1000 in saturation buffer were incubated (50 µl/well) for 30 minutes, at 20°C, under agitation. After additional washings, plates were incubated for 30 min at 20°C under agitation with peroxydase-conjugated streptavidin (Amersham) diluted 1/1000 in saturation buffer (50 µl/well). After washings, revelation was done by adding 50 µl/well of revelation buffer (OPDA 0.4 mg/ml (Sigma) and H₂O₂ 0.05 % in 100mM pH 4.5 citrate buffer) for 20 min at room temperature in the darkness. Revelation was stopped by adding 50 µl/well HCl 1N. Optical densities were read at 490 and 620 nm by using Emax immunoreader (Molecular Devices). Antibody titre were calculated by the 4 parameter mathematical method using SoftMaxPro software.

### 4. Cellular response

Spleen cells were isolated 14d Post II from all groups immunized with FG as well as from naïve mice for use as a negative control for the FG-specific cellular response analysis.

Samples were analysed for both FG-specific lymphoproliferation and cytokine (IFN-g + IL-5) secretion.

Proliferation was evaluated after a 96h incubation of 4x10⁵ cells/well of 96 well plates with 200 µl of media containing 10 to 0.03 µg/ml of FG (3-fold dilutions). Upon ³H-thymidine incorporation, the FG specific proliferation was measured following our standard protocol. Cytokine induction was evaluated after 96 h incubation of 2.5x10⁶ cells per well of 24 well with 1 ml of media containing 10µg to 0.01 µg of FG (10-fold dilutions). Supernatants were then harvested to determine the quantity of IFN-γ and IL-5 induced by ELISA following our standard protocol.

### RESULTS

### 1. GROUPS.

14 groups received two IM immunizations 28 days apart of various formulations containing either the 11-valent conjugated vaccine or FG or a combination of both formulated with alum 3D-MPL, CpG, Cpg alum or alum.

Results presented in Sections 1 to 4 are discussed. In each Section, results of FG Alum are included as a control for the induction of a specific RSV immune response with a predominantly Th2-type adjuvant. A group naïve mice was also added for use as an internal control of FG-specific CMI analysis.

### Section 1:

| Groups | Antigen | Adjuvant | Route |
|---|---|---|---|
| A | PS-PD (0.5 µg) | Alum 3D-MPL | IM |
| B | FG (2µg) | Alum 3D-MPL | IM |
| °C | PS-PD (0.5 µg) + FG (2 µg) | Alum 3D-MPL | IM |
| D | FG (2µg) | Alum | IM |
| E | none | none | IP |

### Section 2:

| Groups | Antigen | Adjuvant | Route |
|---|---|---|---|
| A | PS-PD (0.5 µg) | 3D-MPL + | IM |
| | | QS21 | |
| B | FG (2 µg) | 3D-MPL + | IM |
| | | QS21 | |
| C | PS-PD (0.5 µg) + FG (2 µg) | 3D-MPL + | IM |
| | | QS21 | |
| D | FG(2µg) | Alum | IM |
| E | none | none | IP |

### Section 3:

| Groups | Antigen | Adjuvant | Route |
|---|---|---|---|
| A | PS-PD (0.5 µg) | CpG (50 µg) | IM |
| B | FG(2µg) | CpG (50 µg) | IM |
| C | PS-PD (0.5 µg) + FG (2 µg) | CpG (50 µg) | IM |
| D | PS-PD (0.5 µg) | CpG (50 µg) Alum | IM |
| E | FG (2µg) | CpG (50 µg) Alum | IM |
| F | PS-PD (0.5 µg) + FG (2 µg) | CpG (50 µg) Alum | IM |
| G | FG (2µg) | Alum | IM |
| H | none | none | |

### Section 4:

Same groups as for Section 3

### 2. SECTION 1:

### 2.1. Anti-FG antibodies:

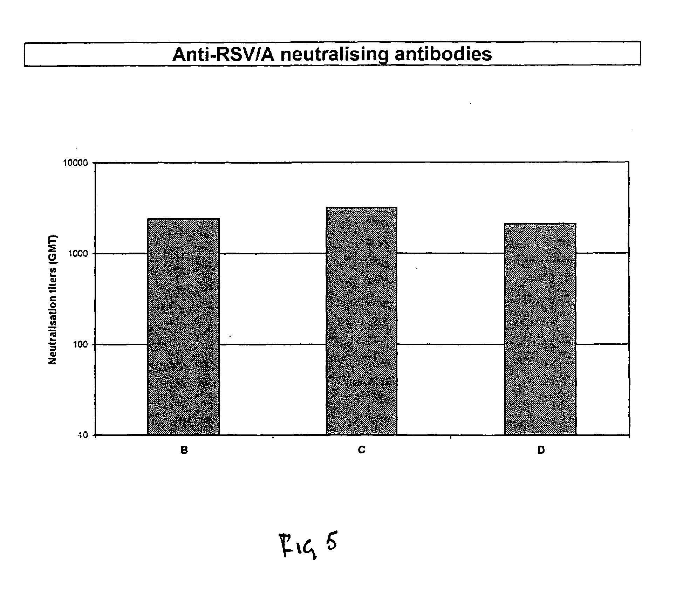

Similar anti-RSVA neutralizing antibody levels were induced by FG / Alum / 3D-MPL or FG / Alum / 3D-MPLcombined with the 11-valent PS conjugate (***Figure 5***)*.* A slight synergestic effect for the induction of RSVA neutralising antibodies can in fact be observed upon combination of both vaccines.

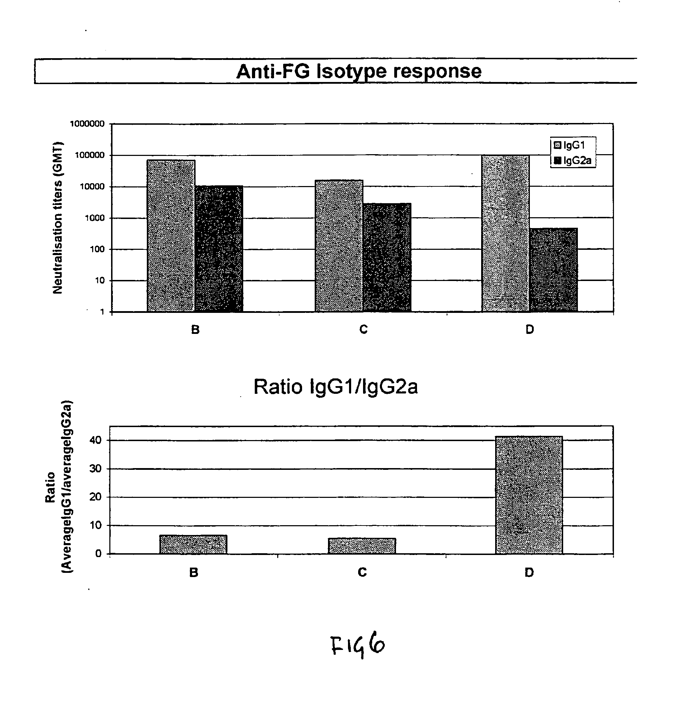

Analysis of individual anti-FG IgG and IgG2a isotype titers showed the induction of similar or slightly lower titers by FG / Alum / 3D-MPL / 11-valent PS conjugate versus FG / Alum / 3D-MPL ***(******Figure 6******)**.*

Analysis of the isotype ratio of IgG2a to IgG1, indicates that the presence of both vaccines does not alter the isotype ratio which remains much lower than the one induced by FG Alum, a recognized Th2 inducing adjuvant.

### 2.2. Anti-Pneumococcal Polysaccharide IgG

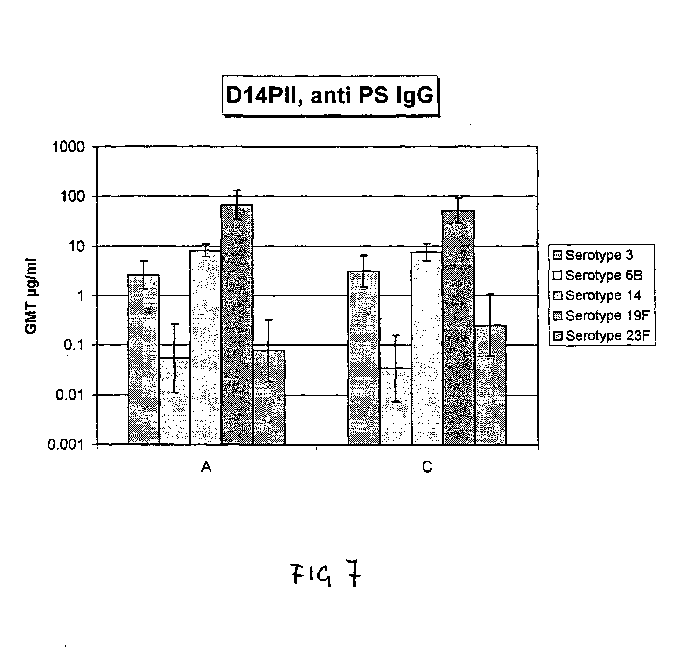

Serum IgG titers to polysaccharides 3, 6B, 14, 19F and 23F were measured as described in Example 1. Similar antibody responses were induced upon immunization with the 11-valent PS conjugate/ Alum / 3D-MPL formulation or with the same vaccine supplemented with FG antigen ***(******Figure 7******).*** This result demonstrated that combining pneumococcal PS conjugates and RSV FG antigen within an Alum 3D-MPL formulation did not reduce or alter the anti-pneumococcal immune response.

### 2.3. FG-specific Cellular response.

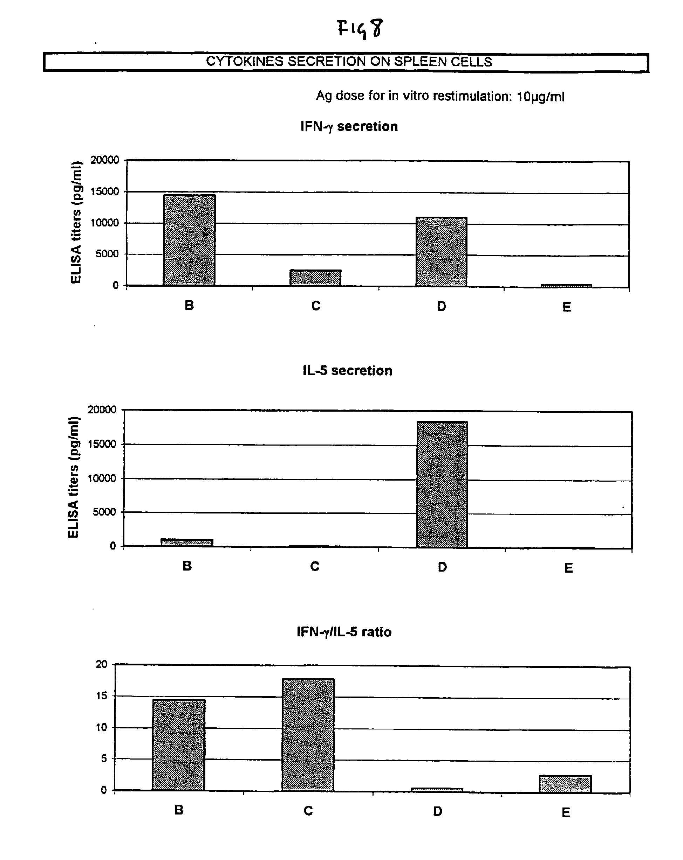

Analysis of the production of IL-5 and IFN-γ suggests that the mixture of FG / Alum / 3D-MPL with the 11-valent PS conjugate does somewhat affect the level of production of these cytokines ***(******Figure 8******)**.* However the ratio of the measured cytokines, an indicator of the Th profile of the response, is not affected by the mixture and remains quite different from the ratio observed for the FG Alum formulation, a Th2 inducing adjuvant.

### 3. SECTION 2: DQ/3D-MPLin

### 3.1. Anti-FG antibodies:

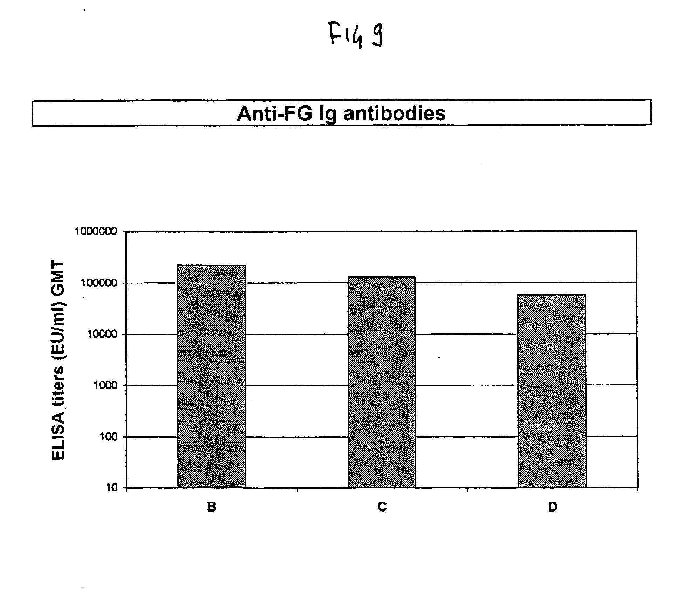

Similar anti-FG Ig antibody levels were induced by FG DQ/3D-MPLin or FG DQ/3D-MPLin combined with the 11-valent PS conjugate ***(******Figure 9******)**.*

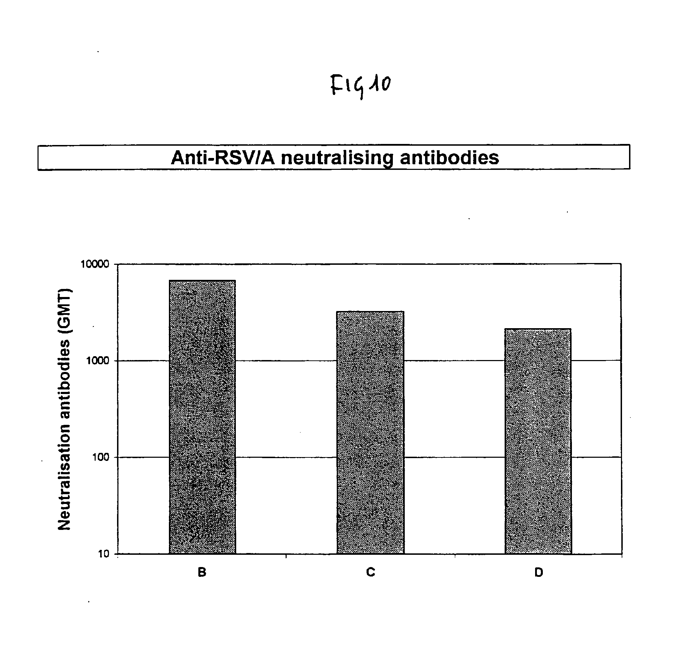

As seen for the anti-FG Ig analysis, no interference is observed on the anti-RSVA neutralizing antibody levels when mixing FG DQ-MPLin with the 11-valent PS conjugate ***(******Figure* 10*****).***

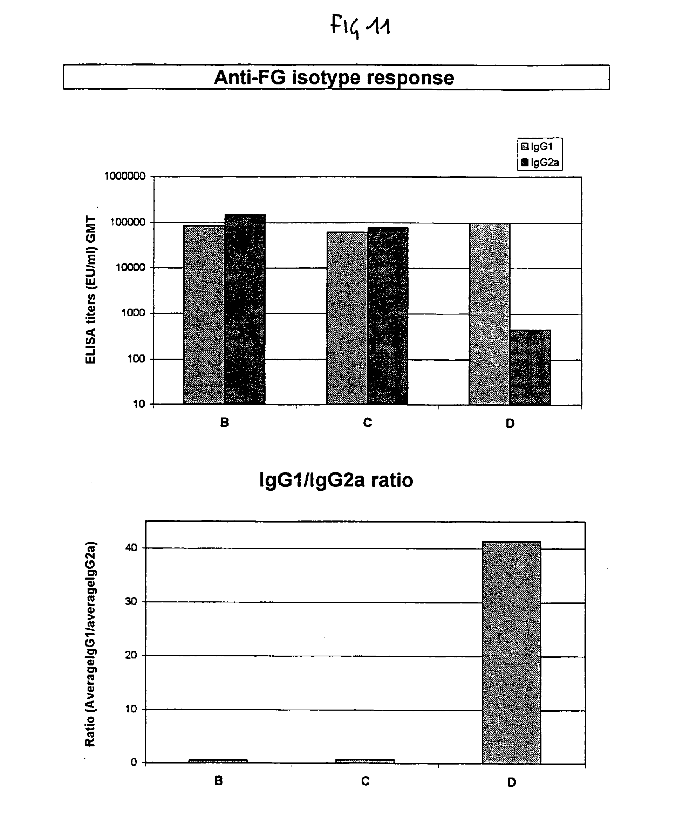

Analysis of individual anti-FG IgG1 and IgG2a isotype titers showed the induction of similar titers by FG DQ-MP/11-valent PS conjugate versus plain FG DQ-MP ***(******Figure 11******)**.* Analysis of the isotype ratio, indicates that the combining of the vaccines does not alter the isotype ratio which remains much lower than the one induced by FG Alum, a recognized Th2 inducing adjuvant.

### 3.2. Anti-Pneumococcal Polysaccharide IgG

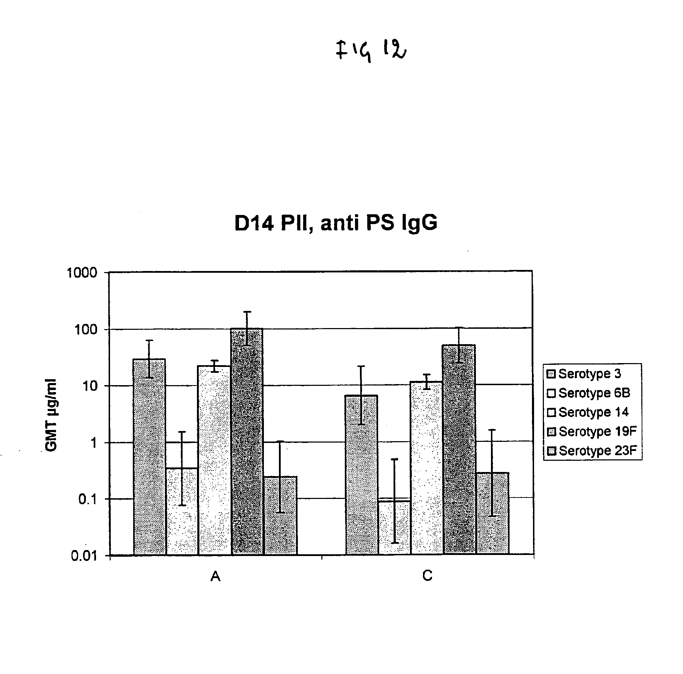

Serum IgG titers to polysaccharides 3, 6B, 14, 19F and 23F were measured as described in Example 1. Similar antibody responses were induced upon immunization with the 11-valent PS conjugate/ DQ/3D-MPLin formulation or with the same vaccine supplemented with FG antigen ***(******Figure 12******)**.* This result demonstrated that combining pneumococcal PS conjugates and RSV FG antigen within a DQ/3D-MPLin formulation did not reduce or alter the anti-pneumococcal immune response.

### 3.3. FG-specific Cellular response.

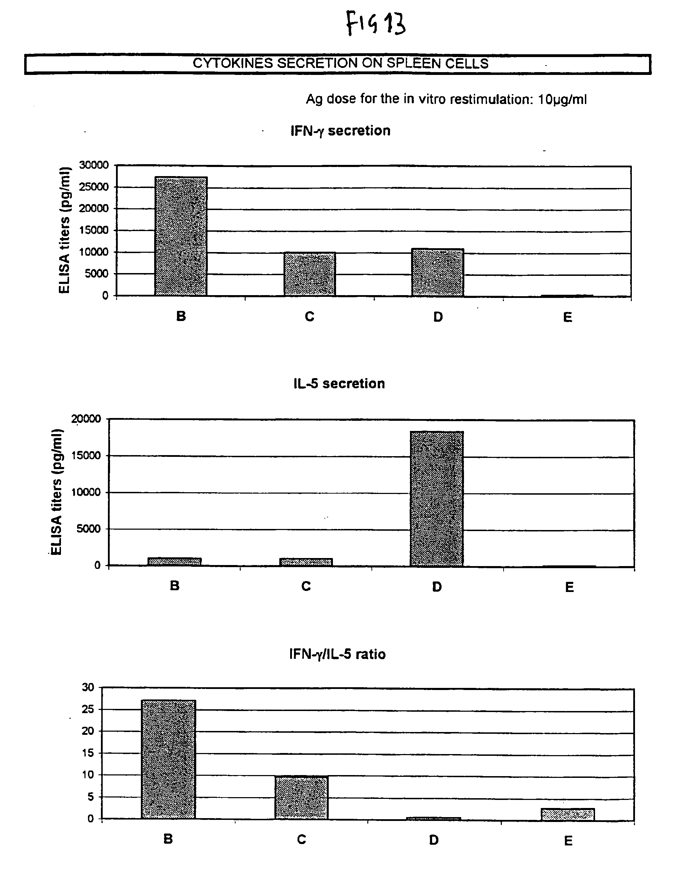

Analysis of the production of IL-5 and IFN-γ initially suggests that the mixture of FG DQ/3D-MPLin with the 11-valent PS conjugate does somewhat affect the level of production of INF-γ *(****Figure 13****).* Further analysis of the ratio of the measured cytokines, indicator of the Th profile of the response, shows that the mixture of FG DQ/3D-MPLin with the 11-valent PS conjugate does somewhat affect the Th profile. However, the predominance of IFN-γ over IL-5 is maintained for the combination of the two antigens with DQ/3D-MPLin and remains much higher than what is observed for FG Alum, a recognized Th2 inducing adjuvant.

### 4. SECTION 3: CPG AND CPG ALUM.

### 4.1. Anti-FG antibodies:

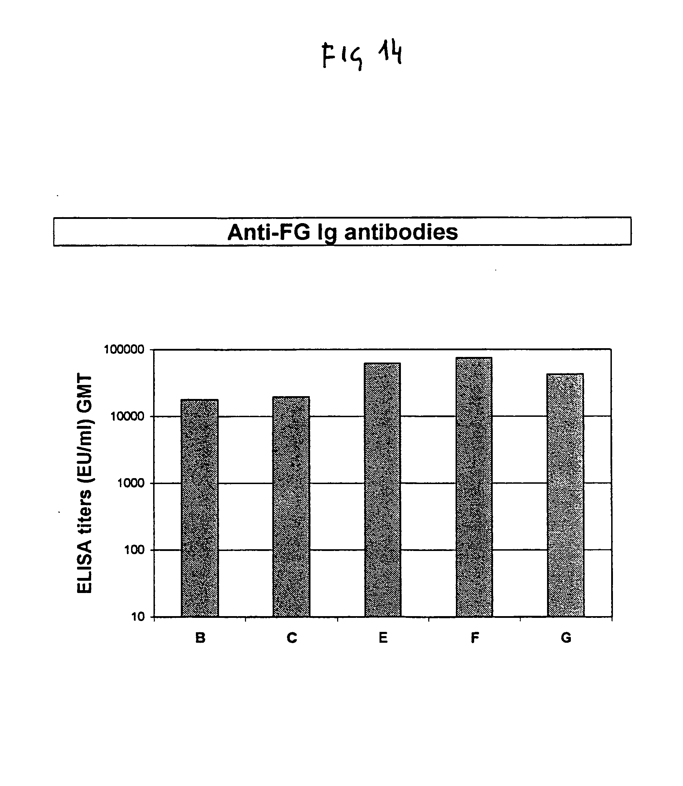

Similar anti-FG Ig antibody levels were induced by FG CpG or FG CpG combined with the 11-valent PS conjugate ***(******Figure* 14****)**. The absence of interference due to the mixture of FG CpG and the 11-valent PS conjugate can also be observed for the CpG alum based formulations.

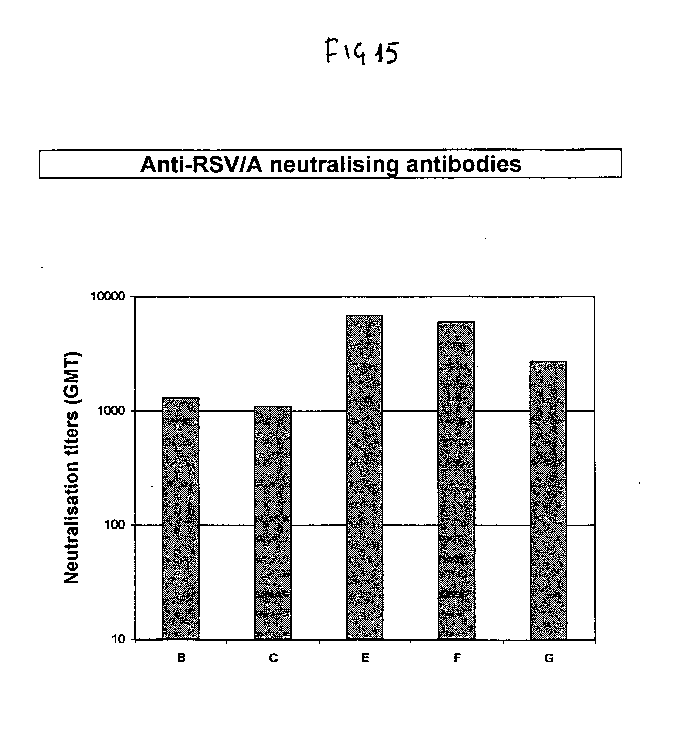

As seen for the anti-FG Ig analysis, no interference is observed on the anti-RSVA neutralizing antibody levels when mixing FG CpG or FG CpG alum with the 11-valent PS conjugate **(*****Figure 15*****).**

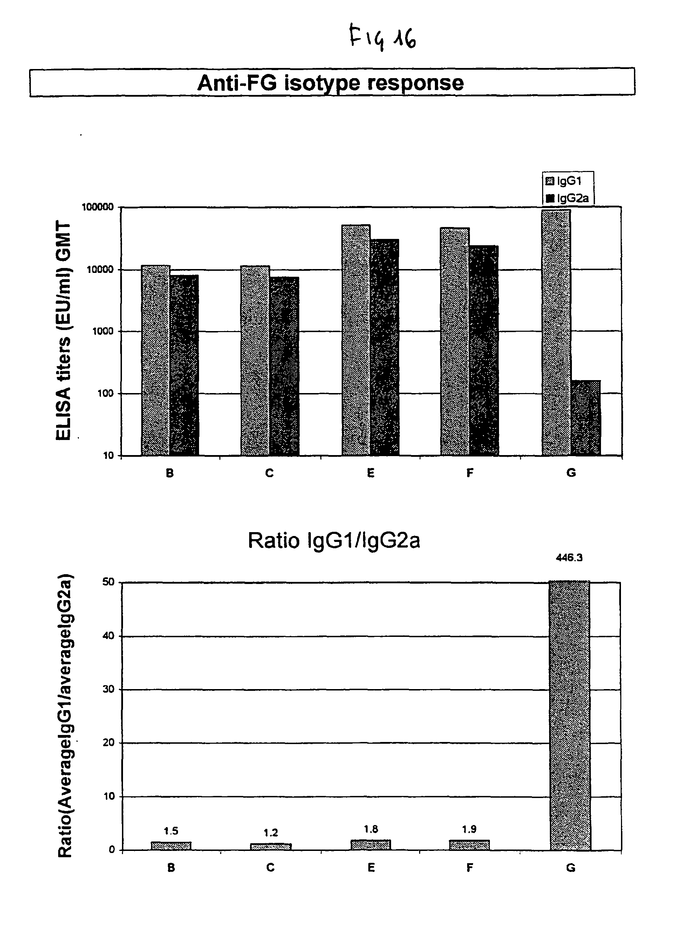

Analysis of individual anti-FG IgG1 and IgG2a isotype titers showed no interference upon addition of the 11-valent PS conjugate to the FG CpG or FG CpG alum formulations ***(******Figure 16******).***

Further analysis of the IgG1/IgG2a isotype ratio, indicates that the mixture of both vaccines does not alter the isotype ratio which remains much lower than the one induced by FG Alum, a Th2 adjuvant.

### 4.2. Anti-Pneumococcal Polysaccharide IgG

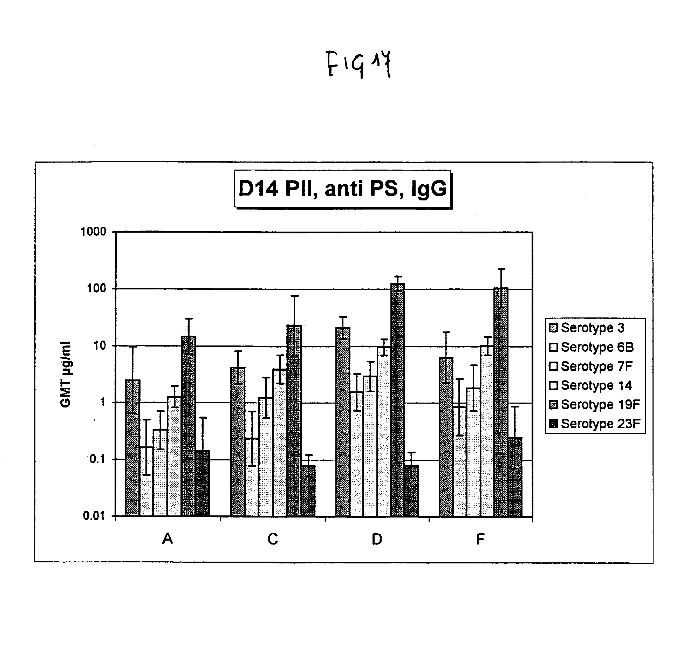

Serum IgG titers to polysaccharides 3, 6B, 7F, 14, 19F and 23F were measured as described in Example 1. In the present example, two adjuvant formulations were tested: CpG and Alum / CpG. For both adjuvant formulations, anti-PS IgG titers were at least similar in animals given the 11-valent PS conjugate supplemented with FG compared to the animals immunized with the 11-valent PS conjugate alone ***(******Figure 17*****)***.* This result demonstrated that combining pneumococcal PS conjugates and RSV FG antigen within a CpG or an Alum CpG formulation did not reduce or alter the anti-pneumococcal immune response. Antibody titers to serotypes 7F and 14 were even significantly improved in the presence of FG using the CpG adjuvant formulation.

### 4.3. FG-specific Cellular response.

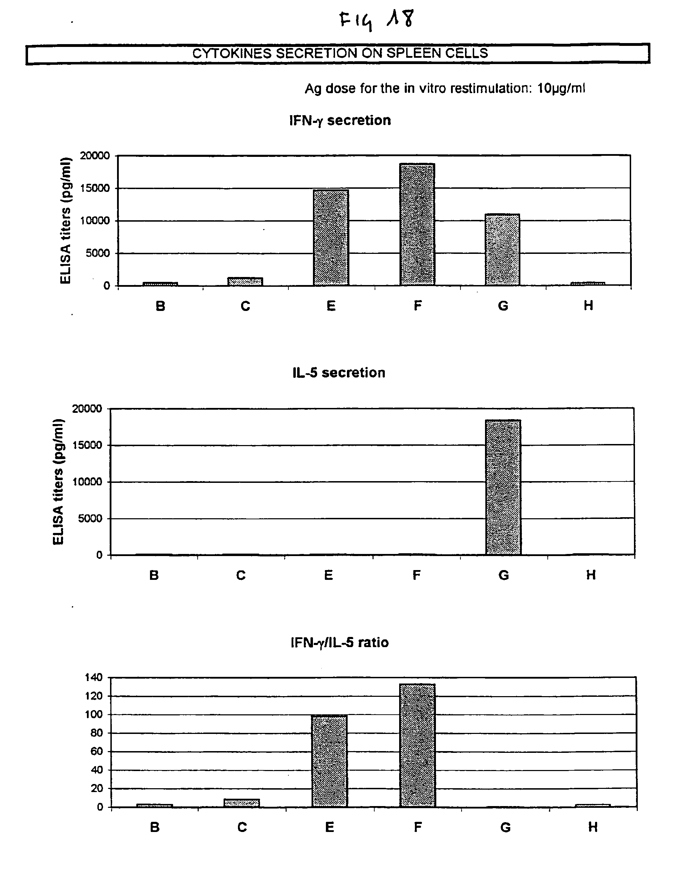

Analysis of the production of IL-5 and IFN-γ suggests that the mixture of FG CpG or CpG alum and the 11-valent PS conjugate does not affect the level of production of these cytokines nor their IFN-γ / IL-5 ratio **(****Figure 18****)**. Therefore, the predominance of IFN-γ, a marker of a Th1 response, is maintained for both combos within the CpG and CpG alum formulations. IFN-γ induction was even improved in the presence of FG using the CpG adjuvant formulation.

### 5. SECTION 4:

The impact of adding FG into PS conjugate formulations on the IgG immune response to protein D (PD) carrier was assessed. PD is a protective antigen against Non Typable *Haemophilus influenzae* B (NTHi) infection. A Th1 type cell-mediated immunity is probably involved in the protection against NTHi. Therefore, anti-PD IgG2a antibodies, which are a good indicator of a Th1 response, were also tested.

### 5.1. Anti-FG antibodies:

See Section 3

### 5.2. Anti-Pneumococcal Polysaccharide IgG

See Section 3

### 5.3. FG-specific Cellular response.

See Section 3

### 5.4. ELISA dosage of anti-PD serum IgG, IgG1, 2a and 2b.

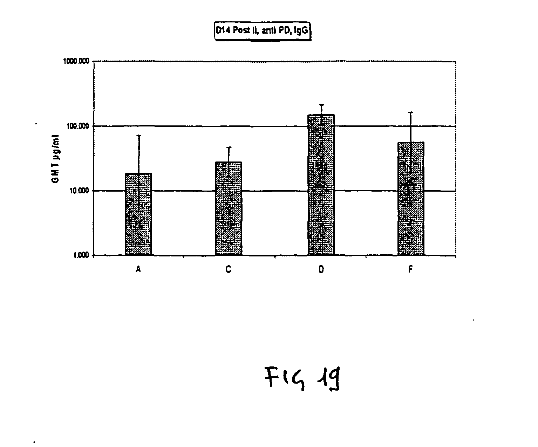

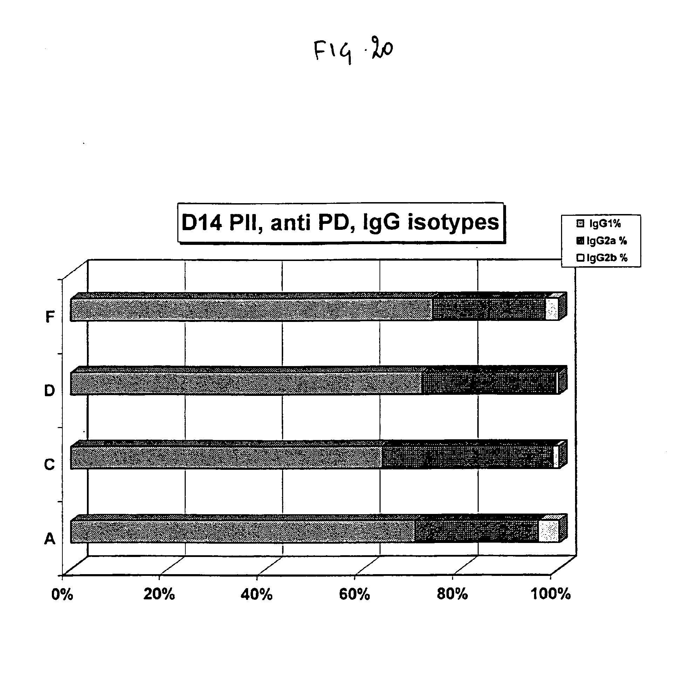

As shown in ***Figure 19****,* total serum IgG responses to PD carrier were not significantly affected by the presence of FG into pneumococcal PS conjugate formulations, whatever the adjuvant used (CpG or Alum CpG). The high proportion of serum anti-PD IgG2a antibodies (around 30 % of total IgG) elicited upon vaccination with CpG or Alum CpG PS conjugate formulations was not modified by the addition of FG into those formulations ***(******Figure 20******)**.* Such an IgG2a response was indicative of a strong Th1 immunity. Taken together, These results demonstrated that combining PD-conjugated pneumococcal PS and RSV FG antigen within a CpG or an Alum CpG formulation did not alter the anti-PD immune response.

### DISCUSSION AND CONCLUSION

### Section 1: Alum / 3D-MPL

The results demonstrated that combining pneumococcal PS conjugates and RSV FG antigen within an Alum 3D-MPL formulation does not alter the humoral anti-pneumococcal immune responses. Both the level of humoral and cellular FG-specific immune responses seem to be slightly affected by the mixture, however not affecting the Th profile of the plain FG formulation.

### Section 2: DQ/3D-MPLin

The results demonstrated that combining pneumococcal PS conjugates and RSV FG antigen in a formulation with DQ/3D-MPLin does not alter the humoral anti-pneumococcal immune responses or the humoral FG-specific responses. Further analysis of the cellular response suggest that the combination of both antigens in a formulation with DQ/3D-MPLin does somewhat affect the induction of IFN-γ. However, this is not affecting the Th profile compared to the FG (alone) formulation.

### Section 3: CpG /CpG Alum

The result demonstrated that combining pneumococcal PS conjugates and RSV FG antigen in a formulation with CpG or Alum CpG did not reduce or alter the humoral anti-pneumococcal immune response or the humoral and cellular FG-specific responses.

### Section 4: CpG /CpG Alum

The results demonstrated that combining PD-conjugated pneumococcal PS and RSV FG antigen in a formulation with CpG or Alum CpG did not reduce or alter the anti-PD immune response.

In conclusion, there is a favorable combination of 23-Valent pneumococcal vaccine with RSV FG 3D-MPL in which there is a synergistic effect for both vaccines in that both vaccines show improvements in certain immunological tests, and no inhibition in any tests. From the above presented results the favorable combination is also observed when combining an 11-valent PD-conjugated pneumococcal PS with RSV FG antigen within a set of Th1 adjuvants. Indeed, for most immuno read-outs for the various Th1 adjuvants tested, the combination of vaccine antigens does not hamper, or only slightly (and not signigicantly so far as the overall response is concerned), the pre-existing characteristics of either vaccines. As observed when combining FG with the 23-valent vaccine in Example 1, in some instances one or the other vaccine did show improvements in certain immunological tests.

## Claims

1. A vaccine composition comprising :
(a) one or more Streptococcus pneumoniae polysaccharides either conjugated to a protein or peptide, or non-conjugated : and
(b) a Respiratory syncythial virus (RSV) antigen in conjunction with an adjuvant which is a stimulator of a Th1 type response.

2. A vaccine composition according to claim 1 in which the Streptococcus pneumoniae polysaccharide is conjugated to a protein from the group comprising; Protein D from Haemophilus influenzae B or a fragment thereof, encompassing T-helper epitopes a lipidated version of protein D (lipoprotein D); Tetanus toxin, and Diphteria Toxin.

3. A vaccine composition according to claim 1 or claim 2 which additionally comprises a carrier.

4. A vaccine composition according to any one of claims 1 to 3 wherein said adjuvant is selected from the group consisting of: 3D-MPL of 100 nm or less than 100nm, QS21, a mixture of QS21 and cholesterol, and a CpG oligonucleotide.

5. A vaccine composition according to claim 4 in which the adjuvant comprises 3D-MPL.

6. A vaccine composition according to claim 5, further comprising QS21.

7. A vaccine composition according to any one of claims 1 to 6 in which the Streptococcus pneumoniae polysaccharide is a 23-valent pneumococcal polysaccharide vaccine.

8. A vaccine composition according to any one of claims 1 to 6 in which the Streptococcus pneumoniae polysaccharide is an 11-valent conjugated pneumococcal polysaccharide vaccine.

9. A vaccine composition according to any one of claims 1 to 8 in which the RSV antigen is a human RSV envelope glycoprotein.

10. A vaccine composition according to any one of claims 1 to 9 in which the RSV antigen is chimeric fusion protein (FG) of the RSV Fusion protein F and RSV Glycoprotein G, or a fragment thereof.

11. A vaccine composition according to any one of claims 1 to 10 in which an influenza virus antigen is additionally present.

12. A vaccine composition according to any one of claims 3 to 11 in which the carrier is selected from the group comprising aluminium hydroxide, aluminium phosphate and tocopherol and an oil in water emulsion.

13. A method of preparing a vaccine according to any one of claims 1 to 12 which method comprises admixing a Streptococcus pneumoniae antigen and an RSV antigen with a Th1 inducing adjuvant.

14. A method according to claim 13, wherein said Streptococcus pneumoniae antigen is a polysaccharide conjugated to protein D or a fragment thereof encompassing T-helper epitopes.

15. The use of a Streptococcus pneumoniae antigen and a RSV antigen in the preparation of a vaccine according to any of claims 1 to 12 for the prevention or amelioration of RSV and/or Streptococcus pneumoniae infection in a patient.

## Patentansprüche

1. Impfstoffzusammensetzung, umfassend:
(a) ein oder mehrere Streptococcus pneumoniae-Polysaccharide, entweder an ein Protein oder Peptid konjugiert oder nicht-konjugiert; und
(b) ein Antigen des respiratorischen Syncythialvirus (RSV) zusammen mit einem Adjuvans, das ein Stimulator einer Reaktion des Th1-Typs ist.

2. Impfstoffzusammensetzung gemäß Anspruch 1, worin das Streptococcus pneumoniae-Polysaccharid an ein Protein der Gruppe konjugiert ist, die folgendes umfaßt: Protein D von Haemophilus influenzae B oder ein Fragment davon, das T-Helferepitope umfaßt, eine lipidierte Version von Protein D (Lipoprotein D), Tetanustoxin und Diphtherietoxin.

3. Impfstoffzusammensetzung gemäß Anspruch 1 oder Anspruch 2, die zusätzlich einen Träger umfaßt.

4. Impfstoffzusammensetzung gemäß einem der Ansprüche 1 bis 3, worin das Adjuvans aus der Gruppe ausgewählt ist, die aus folgenden besteht: 3D-MPL von 100 nm oder weniger als 100 nm, QS21, ein Gemisch aus QS21 und Cholesterol und ein CpG-Oligonukleotid.

5. Impfstoffzusammensetzung gemäß Anspruch 4, worin das Adjuvans 3D-MPL umfaßt.

6. Impfstoffzusammensetzung gemäß Anspruch 5, die ferner QS21 umfaßt.

7. Impfstoff zusammensetzung gemäß einem der Ansprüche 1 bis 6, worin das Streptococcus pneumoniae-Polysaccharid ein 23-valenter Pneumococcus-Polysaccharidimpfstoff ist.

8. Impfstoffzusammensetzung gemäß einem der Ansprüche 1 bis 6, worin das Streptococcus pneumoniae-Polysaccharid ein 11-valenter konjugierter Pneumococcus-Polysaccharidimpfstoff ist.

9. Impfstoffzusammensetzung gemäß einem der Ansprüche 1 bis 8, worin das RSV-Antigen ein humanes RSV-Hüllglycoprotein ist.

10. Impfstoffzusammensetzung gemäß einem der Ansprüche 1 bis 9, worin das RSV-Antigen ein chimäres Fusionsprotein (FG) des RSV-Fusionsproteins F und des RSV-Glycoproteins G ist oder ein Fragment davon.

11. Impfstoffzusammensetzung gemäß einem der Ansprüche 1 bis 10, worin ein Influenzavirus-Antigen zusätzlich vorliegt.

12. Impfstoffzusammensetzung gemäß einem der Ansprüche 3 bis 11, worin der Träger aus der Gruppe ausgewählt ist, die Aluminiumhydroxid, Aluminiumphosphat und Tocopherol und eine Öl-in-Wasser-Emulsion umfaßt.

13. Verfahren zum Herstellen eines Impfstoffs gemäß einem der Ansprüche 1 bis 12, wobei das Verfahren das Vermischen eines Streptococcus pneumoniae-Antigens und eines RSV-Antigens mit einem Th1-induzierenden Adjuvans umfaßt.

14. Verfahren gemäß Anspruch 13, worin das Streptococcus pneumoniae-Antigen ein Polysaccharid ist, das an Protein D oder einem Fragment davon, das T-Helferepitope umfaßt, konjugiert ist.

15. Verwendung eines Streptococcus pneumoniae-Antigens und eines RSV-Antigens in der Herstellung eines Impfstoffs gemäß einem der Ansprüche 1 bis 12 zur Prävention oder Linderung von RSV und/oder einer Streptococcus pneumoniae-Infektion in einem Patienten.

## Revendications

1. Composition de vaccin comprenant .
(a) un ou plusieurs polysaccharides de *Streptococcus pneumoniae* conjugués à une protéine ou à un peptide ou non conjugués ; et
(b) un antigène de virus syncytial respiratoire (VSR) en conjonction avec un adjuvant qui est un stimulateur d'une réponse de type Th1.

2. Composition de vaccin selon la revendication 1, dans laquelle le polysaccharide de *Streptococcus pneumoniae* est conjugué à une protéine du groupe comprenant : la protéine D de *Haemophilus influenzae B* ou un fragment de celle-ci, couvrant des épitopes T-auxiliaire, une version lipidée de la protéine D (lipoprotéine D) ; la toxine tétanique et la toxine diphtérique.

3. Composition de vaccin selon la revendication 1 ou la revendication 2 qui comprend en outre un véhicule.

4. Composition de vaccin selon l'une quelconque des revendications 1 à 3, dans laquelle ledit adjuvant est choisi dans le groupe consistant en : 3D-MPL de 100 nm ou moins de 100 nm, QS21, un mélange de QS21 et de cholestérol, et un oligonucléotide CpG.

5. Composition de vaccin selon la revendication 4, dans laquelle l'adjuvant comprend 3D-MPL.

6. Composition de vaccin selon la revendication 5, comprenant en outre QS21.

7. Composition de vaccin selon l'une quelconque des revendications 1 à 6, dans laquelle le polysaccharide de *Streptococcus pneumoniae* est un vaccin 23-valent de polysaccharide pneumococcique.

8. Composition de vaccin selon l'une quelconque des revendications 1 à 6, dans laquelle le polysaccharide de *Streptococcus pneumoniae* est un vaccin 11-valent conjugué de polysaccharide pneumococcique.

9. Composition de vaccin selon l'une quelconque des revendications 1 à 8, dans laquelle l'antigène du RSV est une glycoprotéine d'enveloppe de RSV humain.

10. Composition de vaccin selon l'une quelconque des revendications 1 à 9, dans laquelle l'antigène du RSV est une protéine de fusion chimérique (FG) de la protéine de fusion F du RSV et de la glycoprotéine G du RSV, ou un fragment de celle-ci.

11. Composition de vaccin selon l'une quelconque des revendications 1 à 10, dans laquelle un antigène de virus *influenza* est en outre présent.

12. Composition de vaccin selon l'une quelconque des revendications 3 à 11, dans laquelle le véhicule est choisi dans le groupe comprenant l'hydroxyde d'aluminium, le phosphate d'aluminium et le tocophérol et une émulsion huile dans l'eau.

13. Procédé de préparation d'un vaccin selon l'une quelconque des revendications 1 à 12, lequel procédé comprend le mélangeage d'un antigène de *Streptococcus pneumoniae* et d'un antigène du RSV avec un adjuvant inducteur Th1.

14. Procédé selon la revendication 13, dans lequel ledit antigène de *Streptococcus pneumoniae* est un polysaccharide conjugué à la protéine D ou à un fragment de celle-ci couvrant des épitopes de T-auxiliaire.

15. Utilisation d'un antigène de *Streptococcus pneumoniae* et d'un antigène du RSV dans la préparation d'un vaccin selon l'une quelconque des revendications 1 à 12 pour la prévention ou l'amélioration de l'infection par le RSV et/ou par *Streptococcus pneumoniae* chez un patient.
